(19) Europäisches Patentamt
European Patent Office
Office européen des brevets

(11) **EP 4 763 079 A1**

(12) **EUROPEAN PATENT APPLICATION**
published in accordance with Art. 153(4) EPC

(43) Date of publication:
**24.06.2026 Bulletin 2026/26**

(21) Application number: **23963053.6**

(22) Date of filing: **26.12.2023**

(51) International Patent Classification (IPC):
**A61B 5/1455** (2006.01)

(52) Cooperative Patent Classification (CPC):
**A61B 5/1455**

(86) International application number:
**PCT/JP2023/046667**

(87) International publication number:
**WO 2025/141699 (03.07.2025 Gazette 2025/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Sigmakoki Co., Ltd.**
**Saitama 350-1297 (JP)**

(72) Inventors:
• **MORIO, Kouichi**
**Tokyo 130-0021 (JP)**
• **EJIMA, Satoshi**
**Tokyo 130-0021 (JP)**

(74) Representative: **Müller-Boré & Partner**
**Patentanwälte PartG mbB**
**Friedenheimer Brücke 21**
**80639 München (DE)**

(54) **INTRAVASCULAR PLASMA INDEX MEASUREMENT SYSTEM**

(57) [Problem]
Provided is an intravascular plasma index measurement system capable of detecting, with high accuracy, an intravascular plasma index that serves as an indicator of a deficiency of water required within the body even in a case where fluctuations in a pulse wave are large.
[Solution]
An intravascular plasma index measurement system includes: first irradiation means that irradiates a living body with a first light; first light receiving means that receives the first light emitted from the first irradiation means and transmitted through or reflected by the living body; first calculation means that calculates a measurement value $C$ related to an amplitude of the first light for $T_1$ seconds longer than a heartbeat cycle of the living body based on the first light received by the first light receiving means; second calculation means that calculates an average value $C_{AV}$ related to the amplitude of the first light for $T_2$ seconds longer than the $T_1$ seconds based on the first light received by the first light receiving means; and third calculation means that calculates an intravascular plasma index $M$ related to intravascular plasma of the living body based on the measurement value $C$ calculated by the first calculation means and the average value $C_{AV}$ calculated by the second calculation means.

Fig. 4

## Description

### TECHNICAL FIELD

The present invention relates to an intravascular plasma index measurement system capable of non-invasively, easily, and accurately measuring the proportion of plasma in intravascular blood (hereinafter referred to as a plasma fraction), and calculating an intravascular plasma index that serves as an indicator of dehydration based on the plasma fraction.

### BACKGROUND ART

Conventionally, a large number of accidents caused by dehydration have occurred. Fluid intake is indispensable for maintaining human life. In general, fluid intake is voluntarily performed by living organisms, not limited to humans, based on a subjective symptom of "thirst" that arises from a deficiency of plasma in blood vessels and water, which is a main component of plasma, and a resulting reduction in the amount of water delivered throughout the body by blood. However, when a living organism is in a state of tension or excitement, such subjective awareness may diminish, and particularly under a blazing sun, this may lead to a high possibility of accidents caused by dehydration. In addition, as people grow older, their sensitivity to a water deficiency becomes dulled, and they may fail to take voluntary action to replenish water, resulting in frequent accidents caused by dehydration due to insufficient fluid intake.

Furthermore, when the amount of water in blood vessels decreases, this becomes a factor that causes thrombus formation in the blood vessels, and may result in the onset of serious symptoms. In general, it is said that when 2 to 3% or more of body weight (for a person weighing 60 kg, 1.2 to 1.8 liters) in water is lost, there is a risk to life.

For these reasons, a blood water content detection device as described in Patent Document 1, for example, has been required. Patent Document 1 discloses a blood water content detection device configured to remove noise attributable to a low-frequency component resulting from fluctuations associated with autonomic nervous system activity or the like from a pulse wave detected by a pulse wave detection unit, and to calculate an index that changes in accordance with the water content in blood. Patent Document 2 discloses a device configured to irradiate a living body with light of a plurality of wavelengths and to measure biological information that enables acquisition of the water content of the skin or the like, based on the amount of transmitted or reflected light.

### CITED DOCUMENTS

### PATENT LITERATURE

Patent Document 1: WO 2004/006769
Patent Document 2: JP-A-2019-130070

### DISCLOSURE OF THE INVENTION

### PROBLEMS TO BE SOLVED BY THE INVENTION

In general, pulse waveforms are significantly influenced by body weight and individual differences. In addition, when arrhythmia is present, or when the influence of vibrations generated during walking at a rhythm substantially the same as pulsation is detected as noise by the pulse wave detection unit, it is difficult to remove such noise.

In particular, in Patent Document 1, since the detected pulse wave is subjected to differentiation processing along the time axis, there is a problem in that higher-order frequency components included in arrhythmia, walking, or the like are calculated as large values, making it impossible to accurately calculate the water content and to detect a tendency to dehydration.

Further, in Patent Document 2, detection is limited to the water content of the skin, and there is no disclosure suggesting detection of the intravascular plasma volume. Since the water content of the skin does not lead to improvement of dehydration symptoms, there is a problem in that a tendency to dehydration cannot be detected.

Accordingly, the present invention has been derived in order to solve the above-described problems, and an object thereof is to provide an intravascular plasma index measurement system capable of detecting, with high accuracy, an intravascular plasma index that serves as an indicator of a deficiency of water required within the body even in cases where fluctuations in a pulse wave are large due to noise or the like caused by external disturbances such as arrhythmia or walking, or where a large amount of water is present on the skin due to perspiration or the like or within cells due to edema or the like.

### SOLUTIONS TO THE PROBLEMS

An intravascular plasma index measurement system according to a first invention includes: first irradiation means that irradiates a living body with a first light; first light receiving means that receives the first light emitted from the first irradiation means and transmitted through or reflected by the living body; first calculation means that calculates a measurement value C related to an amplitude of the first light for $T_1$ seconds longer than a heartbeat cycle of the living body based on the first light received by the first light receiving means; second calculation means that calculates an average value $C_{AV}$

related to the amplitude of the first light for $T_2$ seconds longer than the $T_1$ seconds based on the first light received by the first light receiving means; and third calculation means that calculates an intravascular plasma index M related to intravascular plasma of the living body based on the measurement value C calculated by the first calculation means and the average value $C_{AV}$ calculated by the second calculation means.

In the intravascular plasma index measurement system according to a second invention, which is in the first invention, the third calculation means calculates the intravascular plasma index M using an equation (1) below.

$$M = K(C - C_{AV})^n + L \quad (1)$$

Here, K, L, and *n* are constants.

In the intravascular plasma index measurement system according to a third invention, which is in the first invention, the intravascular plasma index M is calculated using an equation (2) below.

$$M = f(C - C_{AV}) \quad (2)$$

Here, f(x) is a function in which a differential value obtained by differentiating f(x) with respect to *x* when *x* is equal to or greater than a constant g is smaller than a differential value when x is smaller than the constant g.

In the intravascular plasma index measurement system according to a fourth invention, which is in the first invention, the first calculation means extracts an alternating-current value $A_1$ that indicates the amplitude of the first light and a direct-current value $D_1$ based on an intensity of the first light based on the first light received by the first light receiving means, and calculates the measurement value C based on the extracted alternating-current value $A_1$ and the extracted direct-current value $D_1$.

In the intravascular plasma index measurement system according to a fifth invention, which is in the fourth invention, the third calculation means calculates the intravascular plasma index M using an equation (3) below.

$$M = K(C - C_{AV})^n + L \quad (3)$$

Here, K, L, and *n* are constants, and C = $A_1/D_1$ is defined.

In the intravascular plasma index measurement system according to a sixth invention, which is in the fourth invention, the third calculation means calculates the intravascular plasma index M using an equation (4) below.

$$M = f(C - C_{AV}) \quad (4)$$

Here, f(x) is a function in which a differential value obtained by differentiating f(x) with respect to *x* when *x* is equal to or greater than a constant g is smaller than a differential value when x is smaller than the constant g.

An intravascular plasma index measurement system according to a seventh invention includes: first irradiation means that irradiates a living body with a first light; first light receiving means that receives the first light emitted from the first irradiation means and transmitted through or reflected by the living body; first calculation means that extracts an alternating-current value $A_1$ that indicates an amplitude of the first light and a direct-current value $D_1$ based on an intensity of the first light based on the first light received by the first light receiving means; second irradiation means that irradiates the living body with a second light having a wavelength that is shorter than a wavelength of the first light and at which an absorption characteristic of water is lower than at the wavelength of the first light; second light receiving means that receives the second light emitted from the second irradiation means and transmitted through or reflected by the living body; second calculation means that extracts an alternating-current value $A_2$ that indicates an amplitude of the second light and a direct-current value $D_2$ based on an intensity of the second light based on the second light received by the second light receiving means; and third calculation means that calculates an intravascular plasma index M related to intravascular plasma of the living body based on the alternating-current value $A_1$, the alternating-current value $A_2$, the direct-current value $D_1$, and the direct-current value $D_2$ calculated by the first calculation means and the second calculation means.

In the intravascular plasma index measurement system according to an eighth invention, which is in the seventh invention, the third calculation means calculates the intravascular plasma index M using an equation (5) and an equation (6) below.

$$C = (A_1/D_1)/(A_2/D_2) \quad (5)$$

$$M = K(C - C_{AV})^n + L \quad (6)$$

Here, K, $C_{AV}$, L, and n are constants.

In the intravascular plasma index measurement system according to a ninth invention, which is in the seventh invention, the third calculation means calculates the intravascular plasma index M using an equation (7) and an equation (8) below.

$$C = (A_1/D_1)/(A_2/D_2) \quad (7)$$

$$M = K(C - C_{AV})^n + L \quad (8)$$

Here, K, L, and *n* are constants, and $C_{AV}$ is an

average value of $(A_1/D_1)/(A_2/D_2)$.

In the intravascular plasma index measurement system according to a tenth invention, which is in the seventh invention, the third calculation means calculates the intravascular plasma index M using an equation (9) and an equation (10) below.

$$C = (A_1/D_1)/(A_2/D_2) \quad (9)$$

$$M = f(C - C_{AV}) \quad (10)$$

Here, f(x) is a function in which a differential value obtained by differentiating f(x) with respect to x when x is equal to or greater than a constant g is smaller than a differential value when x is smaller than the constant g, and $C_{AV}$ is an average value of $(A_1/D_1)/(A_2/D_2)$ or a constant.

In the intravascular plasma index measurement system according to an eleventh invention, which is in the first invention, the first light has a wavelength within any range of 960 nm to 980 nm, 1440 nm to 1460 nm, and 1930 nm to 1950 nm.

In the intravascular plasma index measurement system according to a twelfth invention, which is in the seventh invention, the first light has a wavelength within any range of 960 nm to 980 nm, 1440 nm to 1460 nm, and 1930 nm to 1950 nm, and the second light has a wavelength of 800 nm or less.

The intravascular plasma index measurement system according to a thirteenth invention, which is in the first invention, further includes any one or more of: display means that displays the intravascular plasma index M calculated by the third calculation means; vibration means that generates a vibration based on the intravascular plasma index M calculated by the third calculation means; communication means that transmits the intravascular plasma index M calculated by the third calculation means to an external device; and sound means that generates a sound based on the intravascular plasma index M calculated by the third calculation means.

The intravascular plasma index measurement system according to a fourteenth invention, which is in the first invention, further includes: acquisition means that acquires temperature information related to a temperature; and determination means that determines an abnormality of the living body based on the temperature information acquired by the acquisition means and the intravascular plasma index M calculated by the third calculation means.

The intravascular plasma index measurement system according to a fifteenth invention, which is in the first invention, further includes: acquisition means that acquires oxygen information related to blood oxygen saturation of the living body; and determination means that determines an abnormality of the living body based on the oxygen information acquired by the acquisition means and the intravascular plasma index M calculated by the third calculation means.

In the intravascular plasma index measurement system according to a sixteenth invention, which is in the first invention, the first light receiving means receives a first external light different from the first light, and the first calculation means corrects the measurement value C based on the first external light received by the first light receiving means.

In the intravascular plasma index measurement system according to a seventeenth invention, which is in the seventh invention, the first light receiving means receives a first external light different from the first light, the first calculation means corrects any one or more of the alternating-current value $A_1$ and the direct-current value $D_1$ based on the first external light received by the first light receiving means, the second light receiving means receives a second external light different from the second light, and the second calculation means corrects any one or more of the alternating-current value $A_2$ and the direct-current value $D_2$ based on the second external light received by the second light receiving means.

The intravascular plasma index measurement system according to an eighteenth invention, which is in the first invention, further includes: acquisition means that acquires pulse information related to a pulse rate of the living body; and determination means that determines an abnormality of the living body based on the pulse information acquired by the acquisition means and the intravascular plasma index M calculated by the third calculation means.

The intravascular plasma index measurement system according to a nineteenth invention, which is in the first invention, further includes: acquisition means that acquires any one or more of temperature information related to a temperature, oxygen information related to blood oxygen saturation of the living body, and pulse information related to a pulse rate of the living body; and determination means that determines a type of water prescribed to the living body based on any one or more of the temperature information, the oxygen information, and the pulse information acquired by the acquisition means, and the intravascular plasma index M calculated by the third calculation means.

In the intravascular plasma index measurement system according to a twentieth invention, which is in the eighth invention, the third calculation means acquires attribute information related to an attribute of the living body, and sets the $C_{AV}$ based on the acquired attribute information.

EFFECTS OF THE INVENTION

According to the first invention to the twentieth invention, the intravascular plasma index M is calculated based on the measurement value C and the average value of the measurement value C for several minutes or more or the constant $C_{AV}$. In a pulse of blood in which the plasma volume is reduced compared to normal, while the amplitude of a signal of a light having a wavelength at which the light is absorbed by water, which is a main component of plasma, decreases, the amplitude of a signal of a light having a wavelength at which the light is not absorbed by water in plasma does not change. Therefore, for example, by calculating the ratio or the difference between the measurement value C and the average value or the constant $C_{AV}$, the intravascular plasma index M can be detected. Here, the measurement value C is measured for $T_1$ seconds longer than the heartbeat cycle of the living body, and this allows detecting, with high accuracy, the intravascular plasma index M even in a case where fluctuations in a pulse wave are large due to noise or the like caused by external disturbances such as arrhythmia or walking.

According to the first invention to the twentieth invention, the intravascular plasma index M is calculated based on the measurement value C and the average value of C for several minutes or more or the constant $C_{AV}$ without being affected by water on the skin surface or water inside cells. The intravascular plasma index M is the proportion of plasma within blood vessels. Water is mainly supplied from plasma in the blood to all parts of the body, and a dehydrated state is equivalent to a condition in which intravascular plasma is deficient. Therefore, by accurately calculating the intravascular plasma index M, it becomes possible to more accurately notify a user of the dehydration status and to more easily alert the user to the dangerous condition.

Especially, according to the second invention, the intravascular plasma index M is calculated using the equation (1). Accordingly, when the difference between the measurement value C and the average value $C_{AV}$ is very small, the change in the value of the intravascular plasma index M becomes small because the dehydration symptoms are minor, and when the difference between the measurement value C and the average value $C_{AV}$ becomes large, the change in the value of the intravascular plasma index M becomes large, thereby allowing clearly indicating the features of the dehydration symptoms. Therefore, it becomes possible to more accurately notify the user of the dehydration status and to more easily alert the user to the dangerous condition.

Especially, according to the third invention, the third calculation means calculates the intravascular plasma index M using the equation (2). Accordingly, when (C - $C_{AV}$) is larger than the predetermined value g, the change in the intravascular plasma index M becomes small, and when (C - $C_{AV}$) is smaller than the predetermined value g, the change in the intravascular plasma index M becomes large. Therefore, it becomes possible to more accurately notify the user of the dehydration status and to more easily alert the user to the dangerous condition.

Especially, according to the fourth invention, the measurement value C is calculated based on the alternating-current value $A_1$ and the direct-current value $D_1$. The alternating-current value $A_1$ is a value determined by increases and decreases in water, which is a main component in blood plasma, caused by pulsation. Further, the alternating-current value $A_1$ also changes depending on individual differences such as the condition of the body surface, melanin, and fat present outside the blood, as well as on the state of an optical path. Meanwhile, the direct-current value $D_1$ changes depending on the average amount of water contained in intravascular plasma, as well as on individual differences such as the condition of the body surface, melanin, and fat present outside the blood, and on the state of the optical path. Accordingly, for example, by dividing the alternating-current value $A_1$ by the direct-current value $D_1$, the measurement value C representing a dimensionless change in light amount can be obtained. Since this measurement value C is a value corrected for the magnitude of the intrinsic plasma volume in the living body that is not dependent on pulsation, as well as for the influences of the condition of the body surface, fat present outside the blood, and the like, it becomes possible to detect the intravascular plasma index M with higher accuracy.

Especially, according to the fifth invention, the third calculation means calculates the intravascular plasma index M using the equation (3). Accordingly, when the difference between the measurement value C and the average value $C_{AV}$ is very small, the change in the intravascular plasma index M becomes small because the dehydration symptoms are minor, and when the difference between the measurement value C and the average value $C_{AV}$ becomes large, the change in the intravascular plasma index M becomes large, thereby allowing clearly indicating the features of the dehydration symptoms. Therefore, it becomes possible to more accurately notify the user of the dehydration status and to more easily alert the user to the dangerous condition.

Especially, according to the sixth invention, the third calculation means calculates the intravascular plasma index M using the equation (4). Accordingly, when (C - $C_{AV}$) is larger than the predetermined value g, the change in the intravascular plasma index M becomes small, and when (C - $C_{AV}$) is smaller than the predetermined value g, the change in the intravascular plasma index M becomes large. Therefore, it becomes possible to more accurately notify the user of the dehydration status and to more easily alert the user to the dangerous condition.

Especially, according to the seventh invention, the intravascular plasma index M is calculated based on the alternating-current value $A_1$, the alternating-current value $A_2$, the direct-current value $D_1$, and the direct-current value $D_2$. The alternating-current value $A_1$ is a value determined by increases and decreases in water, which is a main component in blood plasma, hemoglobin, and the like, caused by pulsation. Further, the alternating-current value $A_1$ also changes depending on individual differences such as the condition of the body surface, melanin, and fat present outside the blood, as well as on the state of the optical path. Meanwhile, the direct-current value $D_1$ changes depending on the average amount of water contained in intravascular plasma and the average value of hemoglobin, as well as on individual differences such as the condition of the body surface, melanin, and fat present outside the blood, and on the state of the optical path. In contrast, while the alternating-current value $A_2$ is a value determined by increases and decreases in water, which is a main component in blood plasma, hemoglobin, and the like, caused by pulsation, the alternating-current value $A_2$ is less affected by the absorption by water than $A_1$. The alternating-current value $A_2$ also changes depending on individual differences such as the condition of the body surface, melanin, and fat present outside the blood, as well as on the state of the optical path. Meanwhile, the direct-current value $D_2$ changes depending on the average amount of water contained in intravascular plasma and the average value of hemoglobin, as well as on individual differences such as the condition of the body surface, melanin, and fat present outside the blood, and on the state of the optical path, but the direct-current value $D_2$ is less affected by the absorption by water than $D_1$. Accordingly, by performing the calculation for each of the two wavelengths, and calculating the ratio of the two amplitudes, it is possible to appropriately eliminate the influence of extravascular water and biological tissue, and to accurately calculate the intravascular plasma index M.

Especially, according to the eighth invention, the third calculation means calculates the intravascular plasma index M using the equation (5) and the equation (6). Accordingly, when the difference between the measurement value C and the constant $C_{AV}$ is very small, the change in the value of the intravascular plasma index M is shown to be small because the dehydration symptoms are minor, and when the difference between the measurement value C and the constant $C_{AV}$ becomes large, the change in the value of the intravascular plasma index M is shown to be large, thereby allowing clearly indicating the features of the dehydration symptoms. Therefore, it becomes possible to more accurately notify the user of the dehydration status and to more easily alert the user to the dangerous condition.

Especially, according to the ninth invention, the third calculation means calculates the intravascular plasma index M using the equation (7) and the equation (8). Accordingly, even for a particular living body in which the value of $(A_1/D_1)/(A_2/D_2)$ under normal conditions is larger or smaller than that of other living bodies, by defining $C_{AV}$ as the average value of $(A_1/D_1)/(A_2/D_2)$ for that living body, it becomes possible to detect the intravascular plasma index M with higher accuracy.

Especially, according to the tenth invention, the third calculation means calculates the intravascular plasma index M using the equation (9) and the equation (10). Accordingly, when the measurement value C is larger than the predetermined value, the change in the intravascular plasma index M becomes small, and when $(C - C_{AV})$ is smaller than the predetermined value, the change in the intravascular plasma index M becomes large. Therefore, it becomes possible to more accurately notify the user of the dehydration status and to more easily alert the user to the dangerous condition.

Especially, according to the eleventh invention, the first light has a wavelength within any range of 960 nm to 980 nm, 1440 nm to 1460 nm, and 1930 nm to 1950 nm. Accordingly, since the first light has a wavelength near the peak of the absorption coefficient of water, the intravascular plasma index M can be detected with higher accuracy.

Especially, according to the twelfth invention, the first light has a wavelength within any range of 960 nm to 980 nm, 1440 nm to 1460 nm, and 1930 nm to 1950 nm, and the second light has a wavelength of 800 nm or less. Accordingly, since the difference between the absorption coefficient of water at the wavelength of the first light and the absorption coefficient of water at the wavelength of the second light becomes large, the intravascular plasma index M can be detected with higher accuracy.

Especially, according to the thirteenth invention, any one or more of the display means, the vibration means, the communication means, and the sound means are provided. Accordingly, for example, it is possible to notify the living body and the user of an abnormality of the living body using a vibration, a sound, a video, or the like.

Especially, according to the fourteenth invention, the determination means determines an abnormality of the living body based on the temperature information and the intravascular plasma index M. Accordingly, for example, it is possible to determine the abnormality of the living body in consideration of the body temperature of the living body, ambient temperature, and the like.

Especially, according to the fifteenth invention, an abnormality of the living body is determined based on the oxygen information and the intravascular plasma index M. Accordingly, for example, it is possible to determine the abnormality of the living body in consideration of the blood oxygen saturation and the like of the living body. Therefore, for example, even when the measurement value C varies due to an abnormality in hemoglobin oxygen saturation or the like caused by pneumonia or the like, accurate determination is possible.

Especially, according to the sixteenth invention, the first calculation means corrects the measurement value C based on the first external light. Accordingly, for example, since it becomes possible to correct noise caused by the influence of external light when the first light is not emitted, the intravascular plasma index M can be detected with higher accuracy.

Especially, according to the seventeenth invention, the first calculation means corrects any one or more of the alternating-current value $A_1$ and the direct-current value $D_1$ based on the first external light, and the second calculation means corrects any one or more of the alternating-current value $A_2$ and the direct-current value $D_2$ based on the second external light. Accordingly, for example, since it becomes possible to correct noise caused by the influence of external light when the first light and the second light are not emitted, the intravascular plasma index M can be detected with higher accuracy.

Especially, according to the eighteenth invention, an abnormality of the living body is determined based on the pulse information and the intravascular plasma index M. Accordingly, for example, it is possible to determine the abnormality of the living body in consideration of the pulse rate and the like of the living body.

Especially, according to the nineteenth invention, the determination means determines a type of water prescribed to the living body based on any one or more of the temperature information, the oxygen information, and the pulse information, and the intravascular plasma index M. Accordingly, it is possible to determine an appropriate oral rehydration solution or the like corresponding to the condition of the living body.

Especially, according to the twentieth invention, the third calculation means sets $C_{AV}$ based on the attribute information. Accordingly, since it is possible to set a more appropriate $C_{AV}$ in accordance with attributes of the living body such as sex, age, and race, the intravascular plasma index M can be detected with higher accuracy.

BRIEF DESCRIPTION OF THE DRAWINGS

Fig. 1 is a schematic diagram illustrating an exemplary configuration of an intravascular plasma index measurement system according to the embodiment.
Fig. 2 is a schematic diagram illustrating an exemplary configuration of a sensor according to the embodiment.
Fig. 3 is diagrams illustrating an exemplary intravascular plasma index measurement device according to the embodiment.
Fig. 4 is a diagram illustrating functions of an intravascular plasma index measurement device that includes the sensor according to the embodiment.
Fig. 5 is flowcharts illustrating exemplary operations of an intravascular plasma index measurement system 100 according to the embodiment.
Fig. 6(a) is a diagram illustrating a direct-current value $D_1$ and an alternating-current value $A_1$ in first photoelectric data.
Fig. 6(b) is a diagram illustrating a direct-current value $D_2$ and an alternating-current value $A_2$ in second photoelectric data.
Fig. 6(c) is a diagram illustrating the direct-current value $D_1$ and the alternating-current value $A_1$ in the first photoelectric data in a state of water deficiency.
Fig. 6(d) is a diagram illustrating the direct-current value $D_2$ and the alternating-current value $A_2$ in the second photoelectric data in a state of water deficiency.
Fig. 7 is a graph illustrating an intravascular plasma index M relative to (C - CAV).
Fig. 8 is a diagram illustrating light absorption coefficients of $H_2O$ and the like.
Fig. 9 is a flowchart illustrating an operation of setting an average value $C_{AV}$ by the intravascular plasma index measurement system according to the embodiment.
Fig. 10 is a diagram illustrating a flowchart of an operation after the calculation of the intravascular plasma index M in the intravascular plasma index measurement system according to the embodiment.
Fig. 11 is diagrams illustrating exemplary display units 12.

DESCRIPTION OF PREFERRED EMBODIMENTS

The following describes an exemplary intravascular plasma index measurement system according to an embodiment to which the present invention is applied by referring to the drawings.

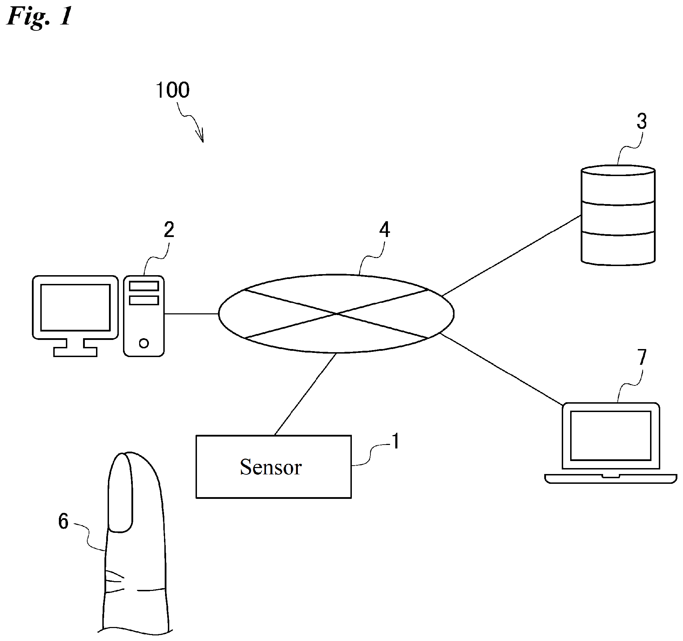

Fig. 1 is a schematic diagram illustrating an exemplary configuration of an intravascular plasma index measurement system 100. In the intravascular plasma index measurement system 100, for example, as illustrated in Fig. 1, an intravascular plasma index measurement device 2, a server 3, a user terminal 7, and a sensor 1 are connected via a public communications network 4. The intravascular plasma index measurement system 100 may include only an intravascular plasma index measurement device 2 that includes the sensor 1.

The server 3 is a storage medium that stores various kinds of data transmitted from the intravascular plasma index measurement device 2 and the sensor 1. The server 3 transmits the various kinds of stored data to the intravascular plasma index measurement device 2, the sensor 1, and the user terminal 7 as necessary. The server 3 may, for example, have at least a part of functions of the intravascular plasma index measurement device 2, and may, for example, perform at least a part of processes instead of the intravascular plasma index measurement device 2.

The public communications network 4 is, for example, an Internet network or the like to which the intravascular plasma index measurement device 2 is connected via a communication circuit. The public communications network 4 may be composed of what is called an optical fiber communications network. The public communications network 4 may be implemented using known communication technologies such as a wired communication network, a wireless communication network, or the like. The user terminal 7 is, for example, owned by a user and the like of a service that uses the intravascular plasma index measurement system 100, and is connected to the intravascular plasma index measurement device 2 via the public communications network 4. The user terminal 7 may be, for example, provided as an electronic device configured to generate a database. For the user terminal 7, for example, an electronic device, such as a personal computer and a tablet terminal, is used. The user terminal 7 may, for example, have at least a part of the functions of the intravascular plasma index measurement device 2. The user terminal 7 may include a display or a speaker (not illustrated) configured to present various kinds of information to the user.

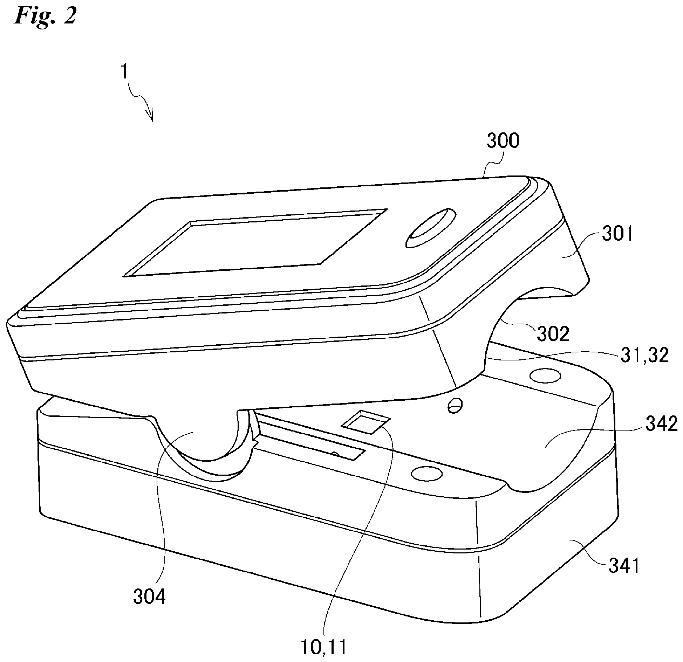

The sensor 1 is a sensor configured to receive a light emitted onto a living body 6. The sensor 1 is a sensor for obtaining an intravascular plasma index M that is an index of plasma in blood vessels by, for example, sandwiching a finger or the like of the living body 6. The structure is appropriate in a case where a light transmitted through a living body is measured. For example, as illustrated in Fig. 2, the sensor 1 includes an infrared light emitting unit 31 that emits a first light, which is an infrared light, a red light emitting unit 32 that emits a second light, which is a red light, an infrared light receiving unit 10 that receives the first light, and a red light receiving unit 11 that receives the second light. The sensor 1 is provided such that the infrared light emitting unit 31 and the red light emitting unit 32 face the infrared light receiving unit 10 and the red light receiving unit 11. The sensor 1 may include, for example, a housing 300 composed of an upper housing 301 having a lower surface on which the infrared light emitting unit 31 and the red light emitting unit 32 are provided, and a lower housing 341 having an upper surface on which the infrared light receiving unit 10 and the red light receiving unit 11 are provided and facing the upper housing 301. The upper housing 301 has the lower surface provided with an upper recess 302 that is recessed upward. The lower housing 341 has the upper surface provided with a lower recess 342 that is recessed downward. The upper recess 302 and the lower recess 342 are formed to allow the finger or the like of the living body 6 to be inserted therebetween. The upper housing 301 and the lower housing 341 are connected to each other via a shaft 304, and formed to be turnable with respect to each other. The upper recess 302 and the lower recess 342 are formed to open in directions away from each other. A spring (not illustrated) is provided between the upper housing 301 and the lower housing 341, and the upper recess 302 and the lower recess 342 are biased in directions in which they close toward each other. The upper housing 301 and the lower housing 341 are formed to hold the finger or the like of the living body inserted between the upper recess 302 and the lower recess 342 in a state of being sandwiched. The sensor 1 may be configured to detect any of temperature information related to an environmental temperature or the temperature of the living body 6, oxygen information related to the blood oxygen saturation of the living body 6, and pulse information related to the pulse rate of the living body 6.

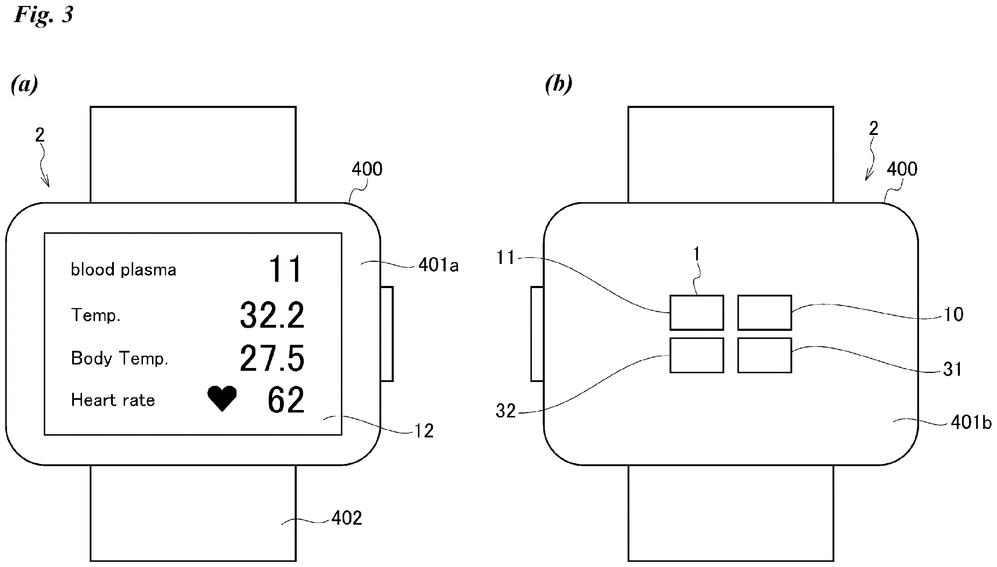

The intravascular plasma index measurement device 2 is a device configured to calculate the intravascular plasma index M. The intravascular plasma index measurement device 2 may be, for example, a wristwatch-type device that is worn on the living body 6 as illustrated in Fig. 3. The intravascular plasma index measurement device 2 may include, for example, a housing 400 having a front surface 401a provided with a display unit 12, such as a monitor. In the intravascular plasma index measurement device 2, for example, the housing 400 may have a back surface 401b provided with the infrared light emitting unit 31, the red light emitting unit 32, the infrared light receiving unit 10, and the red light receiving unit 11, and the sensor 1 may be incorporated. The housing 400 is formed to allow the back surface 401b to be fixed in close contact with the skin of the living body by a wristband 402.

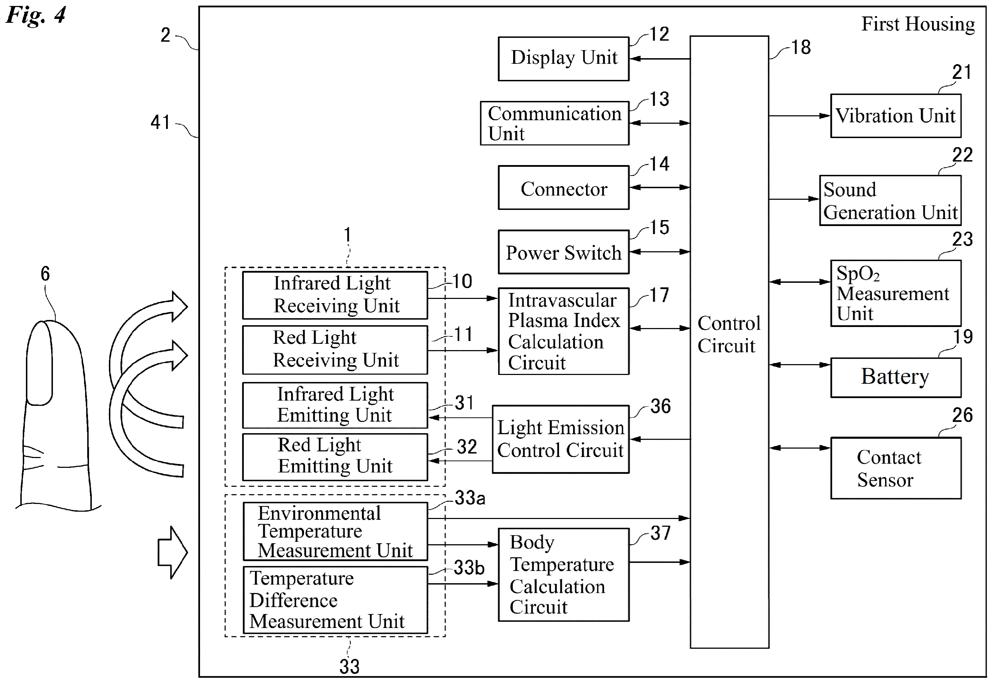

Fig. 4 is a diagram illustrating functions of the intravascular plasma index measurement device 2 that includes the sensor 1. As illustrated in Fig. 4, the intravascular plasma index measurement device 2 includes a first housing 41 in which a control circuit 18 is connected to the display unit 12, a communication unit 13, a connector 14, a power switch 15, an intravascular plasma index calculation circuit 17, a light emission control circuit 36, an environmental temperature measurement unit 33a, a body temperature calculation circuit 37, a vibration unit 21, a sound generation unit 22, an $SpO_2$ measurement unit 23, a battery 19, and a contact sensor 26. The intravascular plasma index measurement device 2 includes the infrared light receiving unit 10 and the red light receiving unit 11 of the sensor 1 connected to the intravascular plasma index calculation circuit 17. The intravascular plasma index measurement device 2 includes the red light emitting unit 32 and the infrared light emitting unit 31 of the sensor 1 connected to the light emission control circuit 36. The intravascular plasma index measurement device 2 includes a temperature measurement unit 33 provided with the environmental temperature measurement unit 33a and a temperature difference measurement unit 33b connected to the body temperature calculation circuit 37.

The infrared light emitting unit 31 emits the first light, which is an infrared light. The infrared light emitting unit 31 emits, for example, an infrared light having a wavelength in the vicinity of 970 nm as the first light. The infrared light emitting unit 31 may emit a light having a wavelength of 1450 nm or 1940 nm at which the absorption coefficient of water exhibits its peak as the first light. The infrared light emitting unit 31 may emit a light having a wavelength within any range of 960 nm to 980 nm, 1440 nm to 1460 nm, and 1930 nm to 1950 nm as the first light. The infrared light emitting unit 31 may emit a light having a plurality of wavelengths including the above-described wavelengths.

The red light emitting unit 32 emits the second light having a wavelength that is shorter than that of the first light and at which an absorption characteristic of water is lower than at the wavelength of the first light. The red light emitting unit 32 emits, for example, a red light having a wavelength of 800 nm or less as the second light. The red light emitting unit 32 emits, for example, a red light having a wavelength in the vicinity of 730 nm as the second light. The red light emitting unit 32 may emit a light having a plurality of wavelengths including the above-described wavelengths.

The infrared light emitting unit 31 and the red light emitting unit 32 may alternately emit the first light and the second light, respectively. The infrared light emitting unit 31 and the red light emitting unit 32 may, for example, repeatedly emit a light for a short time at one-second intervals.

The infrared light receiving unit 10 is a light receiving element that receives the first light emitted from the infrared light emitting unit 31 and transmitted through or reflected by the living body 6, and photoelectrically converts the received light. The infrared light receiving unit 10 outputs an electric signal obtained by photoelectrically converting the first light to the intravascular plasma index calculation circuit 17.

The red light receiving unit 11 is a light receiving element that receives the second light emitted from the red light emitting unit 32 and transmitted through or reflected by the living body 6, and photoelectrically converts the received light. The red light receiving unit 11 outputs an electric signal obtained by photoelectrically converting the second light to the intravascular plasma index calculation circuit 17.

The infrared light receiving unit 10 and the red light receiving unit 11 may be provided with an optical filter that receives only a light having a specific wavelength. For example, the infrared light receiving unit 10 may be provided with an optical filter that receives only a light having a wavelength of 970 nm, and the red light receiving unit 11 may be provided with an optical filter that receives only a light having a wavelength of 730 nm. The infrared light receiving unit 10 and the red light receiving unit 11 may be a single light receiving element configured to receive, for example, a light having a wavelength band of 400 nm to 1000 nm.

The display unit 12 is composed of a liquid crystal display, an electronic display panel, or the like, and displays various kinds of information to the user. The display unit 12 displays, for example, the intravascular plasma index (moisture in vessel) of the living body 6, the environmental temperature (Temp.), the body temperature (Body Temp.) of the living body 6, the pulse rate (Heart rate) of the living body 6, and the oxygen saturation ($SpO_2$). The kinds of information displayed by the display unit 12 include various kinds of information measured related to the living body, such as the intravascular plasma index M of the living body, a pulse rate P of the living body, an environmental temperature Te, and a body temperature T of the living body, but the displayed information is not limited thereto, and various kinds of information, such as the current operation status, the remaining status of the battery 19, and the communication status, may be displayed. The display of the information by the display unit 12 is performed based on the control by the control circuit 18. The display unit 12 is not limited to one that displays characters, and may be, for example, a single red LED (not illustrated) for providing a warning display when the intravascular plasma index M becomes equal to or less than a predetermined value.

The communication unit 13 wirelessly communicates with external devices and various kinds of gateway devices. The communication unit 13 communicates with the server 3 and the user terminal 7, for example, via the public communications network 4, and transmits, for example, the calculated intravascular plasma index M. The communication unit 13 internally includes an antenna that transmits and receives radio waves. The communication unit 13 may include a circuit for converting electric signals transmitted from the control circuit 18 into radio waves, or for converting radio waves received from the external devices into electric signals and transmitting them to the control circuit 18. Information output from the communication unit 13 includes the above-described various kinds of information displayed on the display unit 12.

The connector 14 is an interface circuit for performing wired communication with the external devices. The connector 14 is connected to a wired cable (not illustrated), receives various kinds of information from the external devices, and outputs the received information to the control circuit 18. The connector 14 transmits various kinds of information input from the control circuit 18 to the external devices via the wired cable (not illustrated).

The power switch 15 is composed of a button or the like for turning on or off a power supply of the intravascular plasma index measurement device 2. When the power switch 15 is pressed, a signal transmitted in response to the pressing reaches the control circuit 18. When the control circuit 18 receives the signal transmitted in response to the pressing of the power switch 15, the control circuit 18 performs control for turning on or off the power supply of the intravascular plasma index measurement device 2. The power switch 15 may be configured to turn on or off the power supply of the intravascular plasma index measurement device 2 when the sensor 1 detects the living body 6. The power switch 15 may perform control of turning on or off the power supply, for example, by repeatedly emitting a light for a short time at one-second intervals by the infrared light emitting unit 31 or the red light emitting unit 32 and detecting, for example, the inserted finger of the living body 6 based on an increase or decrease in the amount of the received light.

The intravascular plasma index calculation circuit 17 accepts a signal based on the first light supplied from the infrared light receiving unit 10, and acquires a signal based on the second light supplied from the red light receiving unit 11. The intravascular plasma index calculation circuit 17 has a memory that can store measurement data multiple times at least within one cycle of pulsation of the living body 6. The intravascular plasma index calculation circuit 17 calculates the intravascular plasma index M based on the obtained signal. The intravascular plasma index calculation circuit 17 is configured to retain calculation results and to store an average value and the like calculated over long-term use in the memory.

The intravascular plasma index calculation circuit 17 calculates the intravascular plasma index M of the living body and the pulse rate based on the pulsation of a subject using the signal based on the first light and the signal based on the second light. The intravascular plasma index calculation circuit 17 outputs the measured intravascular plasma index M and the pulse rate of the living body to the control circuit 18.

The control circuit 18 is what is called a central control unit for controlling each component of the intravascular plasma index measurement device 2. The control circuit 18 controls the display unit 12, the communication unit 13, the connector 14, the intravascular plasma index calculation circuit 17, the light emission control circuit 36, the vibration unit 21, the sound generation unit 22, and the $SpO_2$ measurement unit 23. The control circuit 18 includes a central processing unit (CPU), and also includes memories each connected to the CPU. The memories store tables, programs, and the like necessary for controlling hardware resources of the overall intravascular plasma index measurement device 2. The control circuit 18 may execute arithmetic processing of the intravascular plasma index calculation circuit 17.

The battery 19 is a battery for supplying power to the intravascular plasma index measurement device 2, and may be composed of a commercially available button battery or a dry battery. However, the battery 19 is not limited thereto, and may be composed of a rechargeable type battery. The battery 19 is accommodated in a storage pack (not illustrated) that is capable of accommodating the battery 19 along a positive electrode and a negative electrode thereof, and when the power supply is turned on by the power switch 15, electric current flowing through the positive electrode and the negative electrode flows, via the control circuit 18, to the respective components mounted in the intravascular plasma index measurement device 2, thereby enabling operation thereof. Regardless of the operation of the power switch 15, the living body 6 may be photoelectrically detected as described above to turn on the power supply.

The vibration unit 21 generates vibrations under the control by the control circuit 18 when an abnormality occurs in the intravascular plasma index M or the like, thereby outputting a warning signal to the living body 6 or the user. The vibration unit 21 may have, for example, a vibration function, and may operate the vibration function as the warning signal.

The sound generation unit 22 outputs a warning sound, a warning voice, and the like to the living body 6 or the user under the control by the control circuit 18 when an abnormality occurs in the intravascular plasma index M or the like. The sound generation unit 22 may include, for example, a speaker or the like, and may output the warning sound or the warning voice via the speaker.

The $SpO_2$ measurement unit measures oxygen information related to the blood oxygen saturation of the living body 6 by known means under the control by the control circuit 18, and outputs the measured oxygen information to the control circuit 18.

The temperature measurement unit 33 includes the environmental temperature measurement unit 33a and the temperature difference measurement unit 33b. The environmental temperature measurement unit 33a and the temperature difference measurement unit 33b measure temperature information related to the temperature. The environmental temperature measurement unit 33a measures, for example, environmental temperature information related to the environmental temperature Te that is an ambient temperature. The environmental temperature measurement unit 33a measures the environmental temperature information via a thermistor incorporated therein. When the thermistor itself is included inside the intravascular plasma index measurement device 2, the environmental temperature information detected via the thermistor can be regarded as the temperature inside the intravascular plasma index measurement device 2. The environmental temperature measurement unit 33a outputs the detected environmental temperature information to the body temperature calculation circuit 37 and the control circuit 18. The temperature difference measurement unit 33b measures temperature difference information. The temperature difference measurement unit 33b includes a light receiving element that receives an infrared light emitted from the skin as a measurement site of the living body 6. The temperature difference measurement unit 33b measures the temperature difference information based on the received infrared light. The temperature difference information is information related to a difference between the environmental temperature information measured by the environmental temperature measurement unit 33a and the temperature at the measurement site of the living body 6 (skin or the like of the living body). The temperature difference measurement unit 33b outputs the measured temperature difference information to the body temperature calculation circuit 37.

The temperature difference measurement unit 33b is provided, for example, to face a floor or the ground on which the living body 6 is placed so as to receive an infrared light radiated from the floor or the ground, thereby obtaining surrounding temperature conditions in which the living body 6 is placed, and acquiring the intravascular plasma index M, making it possible to determine the level of safety of life of the living body 6.

The body temperature calculation circuit 37 receives the environmental temperature information detected by the environmental temperature measurement unit 33a, and receives the temperature difference information measured by the temperature difference measurement unit 33b. The body temperature calculation circuit 37 obtains the sum of the environmental temperature information and the temperature difference information, and uses the sum as the temperature of the measurement site of the living body 6 as a measurement target. The body temperature calculation circuit 37 converts the calculated temperature information including the information on the body temperature into an axillary body temperature, and outputs it to the control circuit 18. The calculation processes of the temperature measurement unit 33 and the body temperature calculation circuit 37 may be executed by the control circuit 18.

The light emission control circuit 36 outputs a signal for causing the infrared light emitting unit 31 to emit the first light and a signal for causing the red light emitting unit 32 to emit the second light under the control by the control circuit 18.

The contact sensor 26 is composed of a sensor for sensing whether the measurement site of the living body 6 is in contact or not. When the contact of the measurement site of the living body 6 is sensed, the contact sensor 26 notifies the control circuit 18 of the contact, and in a case where the contact sensor 26 does not sense the living body even when the power switch 15 is activated, the emission of light by the light emission control circuit 36 is stopped. The contact sensor 26 is configured to automatically activate the power switch 15 when the contact sensor 26 detects the living body.

The living body 6 is, for example, a human, but is not limited thereto, and the living body 6 may be any animal.

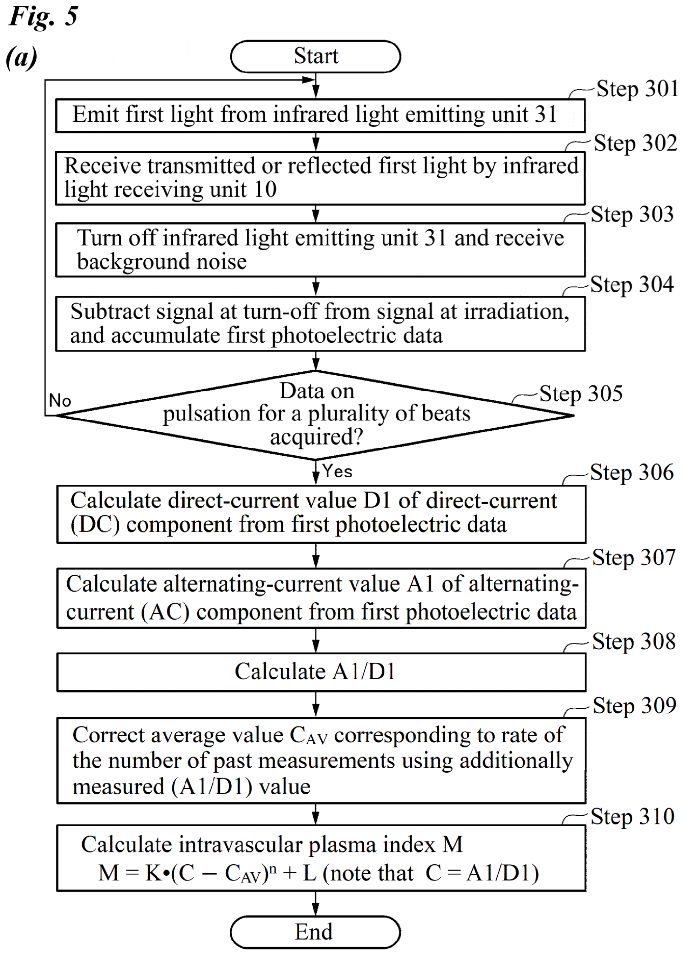

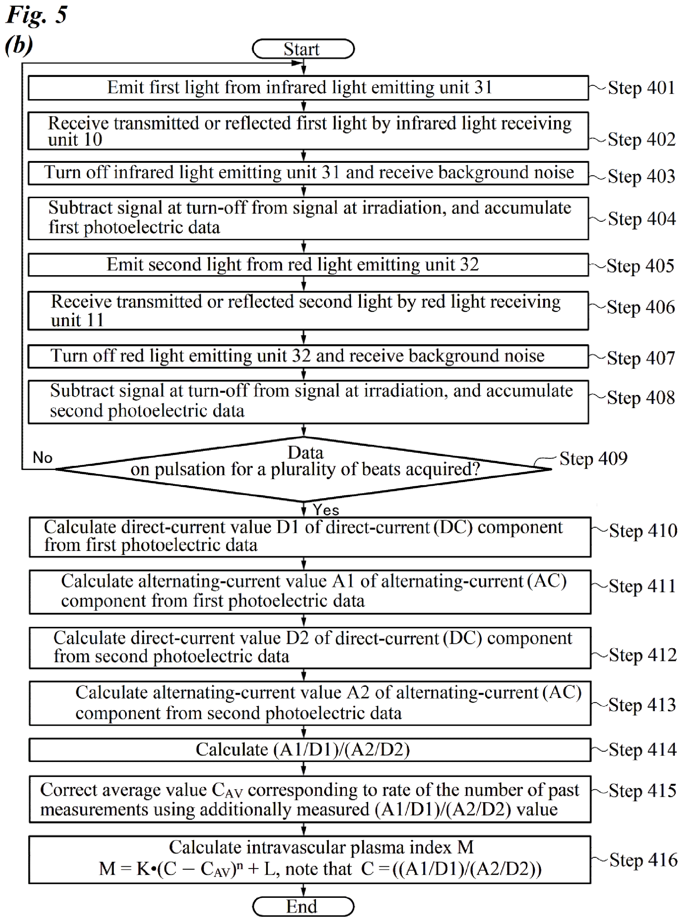

Next, exemplary operations of the intravascular plasma index measurement system 100 according to the embodiment will be described. Fig. 5 is flowcharts illustrating exemplary operations of the intravascular plasma index measurement system 100 according to the embodiment. Fig. 5(a) is a flowchart illustrating an operation of measuring the intravascular plasma index M using the first light.

First, in Step 301, under the control by the control circuit 18, the infrared light emitting unit 31 emits the first light toward the skin of the living body 6. The first light may have a wavelength of any of 1450 nm and 1950 nm at which absorption by water peaks; however, 970 nm is preferable because a wavelength of 1000 nm or less allows a reduction in the size of the light receiving element. In Step 301, the infrared light emitting unit 31 emits the first light toward the skin of the living body 6 for at least $T_1$ seconds or more that is longer than a heartbeat cycle of the living body 6. The infrared light emitting unit 31 may irradiate any site of the living body 6 where blood vessels are present, such as an arm, an ear, or a fingertip, with the first light.

Next, in Step 302, the infrared light receiving unit 10 receives the first light transmitted through or reflected by the living body 6. The received first light is affected by blood pulsation when transmitted through or reflected by the living body 6. Therefore, when a large amount of blood and blood plasma in the blood is flowing, the amount of absorbed light increases due to the influence of water, which is a main component of blood plasma, and the amount of light decreases. When the blood flow is small, light absorption decreases, and the amount of light increases. In Step 302, the infrared light receiving unit 10 receives the first light for at least $T_1$ seconds or more that is longer than the heartbeat cycle of the living body 6. In Step 302, the infrared light receiving unit 10 photoelectrically converts the received first light into an electric signal, and outputs the electric signal of the photoelectrically converted first light to the intravascular plasma index calculation circuit 17.

Next, in Step 303, the infrared light emitting unit 31 turns off the first light, and the infrared light receiving unit 10 receives a first external light that is an external light from inside and outside the room and constitutes noise in a signal. The infrared light receiving unit 10 photoelectrically converts the received first external light into an electric signal, and outputs the electric signal of the photoelectrically converted first external light to the intravascular plasma index calculation circuit 17. The first external light is a light different from the first light, and is a light that does not include the first light emitted from the infrared light emitting unit 31. The first external light is, for example, an external light that the infrared light receiving unit 10 receives when the infrared light emitting unit 31 does not emit the first light.

Next, in Step 304, first photoelectric data obtained by subtracting a value of the photoelectric conversion of the first external light received in Step 303 from a value of the photoelectric conversion of the first light in Step 302 is calculated and accumulated. The steps from Step 301 to Step 304 are preferably performed at a rate of about 130 Hz as a sampling rate so as to avoid integer multiples of 50 Hz and 60 Hz of fluorescent lamps and the like that serve as external light.

Next, in Step 305, the intravascular plasma index calculation circuit 17 determines whether or not the first photoelectric data on pulsation for a plurality of beats has been acquired. In Step 305, the intravascular plasma index calculation circuit 17 determines whether or not the first photoelectric data for at least $T_1$ seconds longer than the heartbeat cycle of the living body 6 has been acquired. For example, since the number of pulsations per minute in a human body is 50 to 100, for example, when data can be acquired 512 times at a sampling rate of 130 Hz, data for at least two or more beats can be obtained, and therefore, the intravascular plasma index calculation circuit 17 determines that acquisition of data 512 times is sufficient. When the data acquisition is insufficient, the process returns to Step 301. When the data acquisition is sufficient, the process proceeds to Step 306. When the measurement of the living body 6 for a long time using the measurement device is allowed, it is preferable to acquire a larger amount of data over a longer period of time in order to acquire more accurate data.

Next, in Step 306, the intravascular plasma index calculation circuit 17 calculates a direct-current value $D_1$ from the first photoelectric data based on the intensity of the first light. The intravascular plasma index calculation circuit 17 calculates and stores the direct-

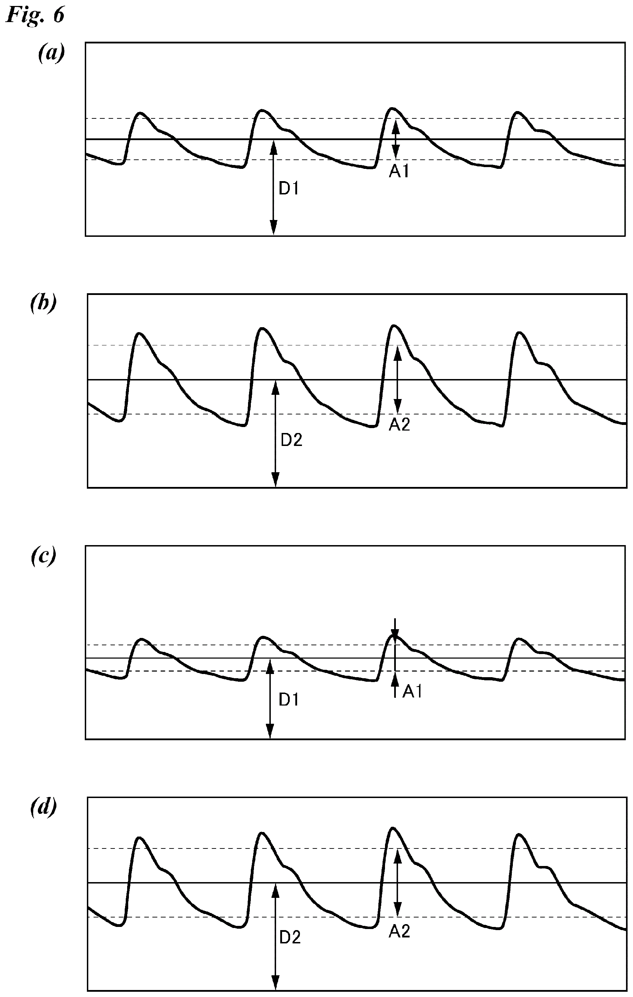

current value $D_1$ that is an average value (DC component) obtained by removing a fluctuation component due to pulsation from the first photoelectric data. Fig. 6(a) is a diagram illustrating the direct-current value $D_1$ and an alternating-current value $A_1$ in the first photoelectric data. Fig. 6(c) is a diagram illustrating the direct-current value $D_1$ and the alternating-current value $A_1$ in the first photoelectric data in a state of water deficiency. For example, as illustrated in Fig. 6(a), the direct-current value $D_1$ is a value obtained by removing an alternating-current component that represents a change in the plasma volume due to pulsation. The direct-current value $D_1$ may be a constant based on the intensity of the first light calculated from the first photoelectric data. The direct-current value $D_1$ may be a value indicating the average of the intensity of the first light calculated from the first photoelectric data. The direct-current value $D_1$ may be, for example, an average value or a root mean square value of the amplitude of the first light. The direct-current value $D_1$ can be obtained, for example, by extracting values corresponding to pulsation for a plurality of times from the acquired first photoelectric data and averaging the extracted values. The direct-current value $D_1$ decreases due to absorption of light by water contained in plasma in blood vessels and living tissue, and by solid components such as melanin. The direct-current value $D_1$ decreases due to water on the skin caused by perspiration. When the average value and the root mean square value are obtained, $T_1$ and $T_2$ need to be accurately calculated for a time period that is an integer multiple of the heartbeat cycle of the living body.

In Step 307, the intravascular plasma index calculation circuit 17 calculates the alternating-current value $A_1$ that indicates the amplitude of the first light from the first photoelectric data. The intravascular plasma index calculation circuit 17 calculates an alternating-current (AC) component that is the fluctuation component due to pulsation, and stores it as the alternating-current value $A_1$. The alternating-current value $A_1$ is a value indicating the amplitude of the pulse of the living body 6. The alternating-current value $A_1$ may be a value indicating the amplitude of the intensity of the first light calculated from the first photoelectric data. The alternating-current value $A_1$ may be an average value of the amplitude of a plurality of pulses. The alternating-current value $A_1$ is calculated, for example, as a root mean square (RMS) value of the first photoelectric data. For example, the alternating-current value $A_1$ may be obtained by subtracting the direct-current value $D_1$ obtained in Step 306 from the first photoelectric data, squaring the subtracted value, further calculating an average value of the squared values over one beat, and taking a square root of the averaged value. In this case, $A_1$ is as illustrated in Fig. 6(a). The alternating-current value $A_1$ may be obtained by calculating a difference between a maximum value and a minimum value in one pulsation and averaging the differences between the maximum values and the minimum values over a plurality of measured pulsations. The alternating-current value $A_1$ may be an amplitude obtained by the Fourier transform. The alternating-current value $A_1$ is affected by a variation in the plasma volume due to pulsation. Therefore, when the plasma volume decreases, since the variation range of plasma due to pulsation decreases, the alternating-current value $A_1$ decreases. The alternating-current value $A_1$ decreases due to absorption of light by plasma in blood vessels and living tissue, and by solid components such as melanin. The alternating-current value $A_1$ decreases due to water on the skin caused by perspiration.

Next, in Step 308, the intravascular plasma index calculation circuit 17 calculates $A_1/D_1$. The intravascular plasma index calculation circuit 17 calculates, for example, $A_1/D_1$ as a measurement value C. The alternating-current value $A_1$ and the direct-current value $D_1$ are attenuated and reduced at a common rate due to factors other than a decrease in light caused by pulsation, namely due to influences of non-pulsating nails, bones, and perspiration of the epidermis, and influences of body tissue, melanin of the skin, and the like. This state is illustrated in Fig. 6(a) and Fig. 6(c). Assume that the amount of melanin in the skin of the living body 6 for which the alternating-current value $A_1$ and the direct-current value $D_1$ as illustrated in Fig. 6(a) have been measured increases due to, for example, suntan. Then, since the amount of light received by the infrared light receiving unit 10 decreases due to the influence of melanin, as illustrated in Fig. 6(c), while the alternating-current value $A_1$ decreases, the direct-current value $D_1$ similarly decreases. Accordingly, by dividing the alternating-current value $A_1$ by the direct-current value $D_1$, a dimensionless number is obtained in which common reduction factors are canceled out. Further, when intravascular plasma decreases, only the alternating-current value $A_1$ decreases, and therefore, $A_1/D_1$ decreases. Accordingly, the measurement value C is a value that is free from the influence of perspiration unrelated to dehydration and the influences of body tissue, melanin of the skin, and the like, from which the influence of noise is removed, and that is correlated with intravascular plasma. When the measured value of $A_1/D_1$ is small, it is difficult to determine whether the reduction in the alternating-current value $A_1$ is caused by weak pulsation of the living body or the reduction in the alternating-current value $A_1$ is caused by a small plasma volume. Here, since it is extremely rare that the living body is in a dehydrated state from the initial stage of use of the housing 400, an average value $C_{AV}$ is acquired over a relatively long period of time, and the intravascular plasma index M is calculated by comparing the measurement value C and the average value $C_{AV}$. This will be described next.

In Step 309, the intravascular plasma index calculation circuit 17 updates the average value of $A_1/D_1$ as the average value $C_{AV}$ by weighting and apportioning the average value of $A_1/D_1$ based on past usage time. The average value $C_{AV}$ is a value related to the average of the amplitude of the first light for $T_2$ seconds longer than

$T_1$ seconds. The average value $C_{AV}$ may be, for example, the average of the value of $A_1/D_1$ for $T_2$ seconds. The average value $C_{AV}$ may be, for example, the average of the value of $A_1/D_1$ for a time period longer than 15 minutes. In Step 309, the intravascular plasma index calculation circuit 17 updates the average value $C_{AV}$ that is the average of $A_1/D_1$ calculated and accumulated since the living body 6 started the use of the intravascular plasma index measurement system 100 based on $A_1/D_1$ calculated in Step 308. Specifically, when the intravascular plasma index measurement device 2 can be worn on the living body 6 for 24 hours like the housing 400, for example, $A_1/D_1$ is measured at a frequency of once per minute, and the value of the average value $C_{AV}$ of $A_1/D_1$ is continuously measured and constantly updated. When the living body 6 that uses the housing 400 changes, the average value $C_{AV}$ may be reset to an initial value. When the housing 300 or the housing 400 is used for the first time after manufacture, or when the average value $C_{AV}$ is reset, a time period until the average value $C_{AV}$ becomes stable (for example, three hours) may be set as $T_2$ seconds, and the average value $C_{AV}$ over $T_2$ seconds may be used as a predetermined constant.

Next, in Step 310, the intravascular plasma index calculation circuit 17 calculates the intravascular plasma index M based on the measurement value C calculated in Step 308 and the average value $C_{AV}$ calculated in Step 309. In Step 310, the intravascular plasma index calculation circuit 17 calculates the intravascular plasma index M based on the difference or the ratio between the measurement value C calculated in Step 308 and the average value $C_{AV}$ calculated in Step 309.

The value of the measurement value C differs among individual living bodies 6, and in particular, there are individual differences due to factors such as the amount of capillaries. On the other hand, since the living body 6 voluntarily takes in water unless it is in a special excited state or the like, the intravascular plasma volume is appropriately maintained for most of the time. Therefore, except for abnormalities in the hemoglobin oxygen saturation caused by pneumonia or the like, the measurement value C takes a substantially uniform value. Accordingly, by examining the difference between the measurement value C and the average value $C_{AV}$, it becomes possible to detect a decrease in intravascular plasma of the living body 6.

In Step 310, the intravascular plasma index calculation circuit 17 calculates the intravascular plasma index M by raising a value of C - $C_{AV}$ to the n-th power, further multiplying the result by a constant K, and adding a constant L, as shown in, for example, an equation (1). For example, the constant K is 10000, and the constant L is 100, but the constants K and L are not limited thereto, and may be any constant.

$$M = K(C - C_{AV})^n + L \quad (1)$$

Here, K, $C_{AV}$, L, and *n* are constants.

In Step 310, the intravascular plasma index calculation circuit 17 calculates the intravascular plasma index M by raising the value of C - $C_{AV}$ to the n-th power, further multiplying the resulting value by the constant K, and adding the constant L, as shown in, for example, an equation (3).

$$M = K(C - C_{AV})^n + L \quad (3)$$

Here, K, L, and *n* are constants, $C_{AV}$ is the average value of $A_1/D_1$, and C = $A_1/D_1$ is defined.

Here, the constant n in the power is, for example, 3, and by cubing the value, it becomes possible to express the intravascular plasma index M such that when there is a very small change in (C - $C_{AV}$) and (C - $C_{AV}$) is small, the resulting value is numerically smaller, while when there is a large change in (C - $C_{AV}$) and (C - $C_{AV}$) is large, the resulting value is larger. The power is not limited to a cube, and the odd power such as the fifth power or the seventh power is preferable. Since *n* is the odd power, even when the value of (C - $C_{AV}$) is negative, it is possible to use the value without changing the negative sign. The constant n does not need to be an integer.

In addition, since the calculated value including a fractional part has poor general recognizability, by multiplying the value by the constant K, it becomes possible to set the intravascular plasma index M to a numerical value having a magnitude that is easy for a person to recognize, for example, a numerical range of -10 to 10. In a normal living body 6, a value obtained by multiplying (C - $C_{AV}$)$^n$ by the constant K is near "0," and becomes negative when plasma is insufficient. Therefore, by setting the constant L to, for example, 100 and adding it to the equation (1), when the intravascular plasma index M is sufficient, a value of 100% or more than 100% is displayed, and when the intravascular plasma index M becomes small, for example, it becomes possible to display an appealing value such as 90%. In order to reduce a load on the control circuit 18, the intravascular plasma index M may be obtained using a twodimensional table corresponding to variables C and $C_{AV}$.

On the other hand, when the value of (C - $C_{AV}$) is near zero, that is, when the measurement value C and the average value $C_{AV}$ are substantially equal, the region is one in which no problem regarding dehydration occurs. Taking into account variations among the living bodies 6 and the fluctuation range over time, this corresponds to a region in which it is not necessary to issue a dehydration alert. In addition, there is a case in which C - $C_{AV}$ becomes positive, for example, when a required amount of water is taken at one time. However, such a situation in which the plasma volume is measured to be higher than normal is, except for special accidents, a situation in which the living body 6 has intentionally ingested water, and therefore similarly corresponds to a region in which the risk of dehydration is low.

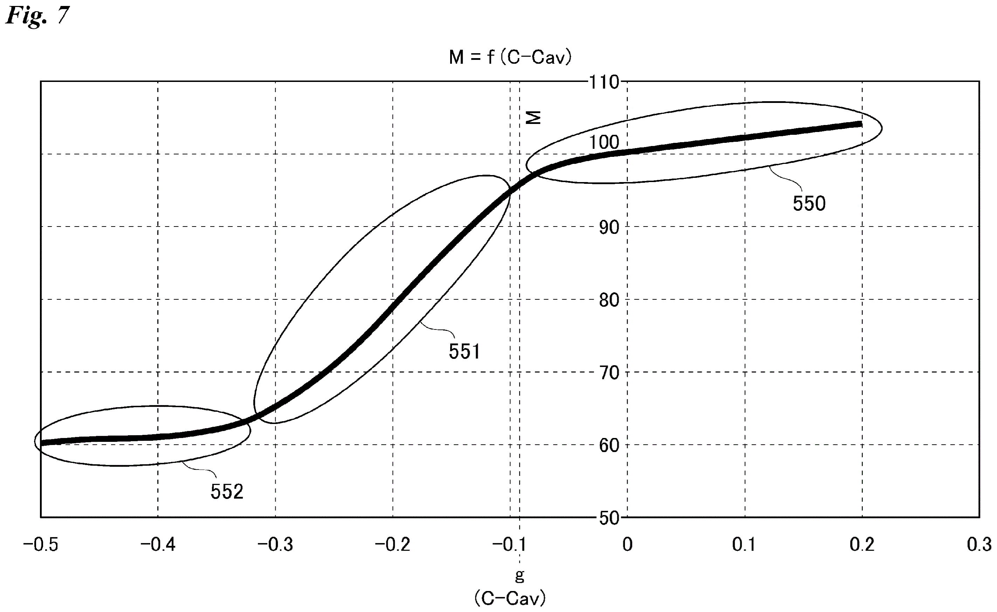

However, when (C - C$_{AV}$) becomes a large negative value, it indicates a dehydrated condition, and therefore, the value of the intravascular plasma index M becomes very important. Fig. 7 is a graph illustrating the intravascular plasma index M on the vertical axis relative to (C - C$_{AV}$) on the horizontal axis. Therefore, as illustrated in Fig. 7, in a first region 550 extending from a negative side to a positive side of a constant g, where (C - C$_{AV}$) is near zero, a change in the intravascular plasma index M may be made small, while in a second region 551 extending largely toward the negative side from the negative side of g, the change in the intravascular plasma index M may be made large. Alternatively, the intravascular plasma index M may be calculated using an equation (2).

$$M = f(C - C_{AV}) \quad (2)$$

Here, f(x) is a function in which a differential value obtained by differentiating f(x) with respect to *x* when *x* is equal to or greater than the constant g, that is, a slope is smaller than a differential value when x is smaller than the constant g. The function f(x) may be, for example, a linear function or a function of second or higher order in which the slope becomes smaller when x exceeds g. For example, g may be a constant near zero, or a constant satisfying -1 < *g* < 1.

As a result, it becomes possible to more accurately notify the user of the dehydration status and to more easily alert the user to the dangerous condition. Note that in a third region 552 in which the value largely shifts toward the negative side, since it is clear that the condition is abnormal, the display may be controlled such that, for example, the intravascular plasma index M does not become equal to or less than a reference value such as 60.

Thus, the operation of measuring the intravascular plasma index M using the first light is completed. By using pulse waves of one beat or more in this manner, it is possible to eliminate a possibility that harmonics caused by arrhythmia or vibrations such as walking are highly observed and noise is measured, which is a concern in a method of differentiating and analyzing pulsation for one cycle, as a result, it becomes possible to calculate a correct proportion of intravascular plasma, and based on this result, the intravascular plasma index M can be obtained more accurately. By detecting an alternating-current component and a direct-current component, it becomes possible to eliminate the influence of an average value of the water amount as well as the influences of individual differences such as the condition of a body surface, melanin, and fat present outside the blood and an optical path, and as a result, the intravascular plasma index M can be obtained more accurately. By using light, the measurement can be performed on the living body 6 in a non-invasive and simple manner. In addition, non-contact measurement is also possible.

Next, an exemplary operation of calculating the intravascular plasma index M using the first light and the second light in the intravascular plasma index measurement system 100 according to the embodiment will be described. Fig. 5(b) is a flowchart illustrating an operation of measuring the intravascular plasma index M using the first light and the second light. Since Steps 401 to 404 are similar to Steps 301 to 304 described above, the explanation is omitted.

In Step 405, under the control by the control circuit 18, the red light emitting unit 32 emits the second light toward the skin of the living body 6. The red light emitting unit 32 irradiates a portion approximately the same as the portion that the infrared light emitting unit 31 irradiated with the first light in Step 301.

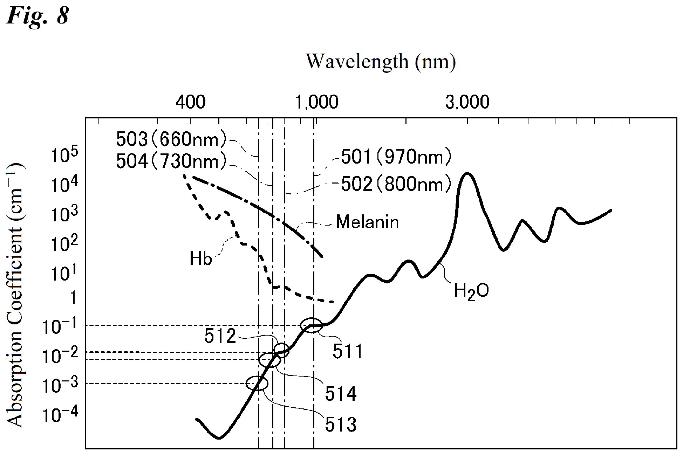

The second light preferably has a wavelength shorter than that of the first light and exhibits a small difference in the light absorption coefficient of water. Fig. 8 is a diagram illustrating light absorption coefficients of H$_2$O and the like. When infrared light of 970 nm indicated by a first dashed line 501 is emitted as the first light, it is preferred to use a wavelength of 800 nm or less indicated by a second dashed line 502 such that the wavelength of the second light exhibit a sufficiently low absorption coefficient of water. The difference in the light absorption coefficient in this case is a difference between a first point 511 that exhibits the absorption coefficient on the first dashed line 501 of 970 nm and a point 512 that exhibits the absorption coefficient on the second dashed line 502 of a wavelength of 800 nm, and the difference is about 10 times. By setting the wavelength of the light radiated from the red light emitting unit 32 to about 660 nm indicated by a third dashed line 503, the light absorption coefficient is further lowered, and therefore, the difference in the absorption coefficient from the first light radiated from the infrared light emitting unit 31 is further increased, thus increasing the accuracy of the calculated value with respect to noise. The difference in light absorption in this case is a difference between the first point 511 that exhibits the absorption coefficient at a wavelength of 970 nm and a third point 513 that exhibits the absorption coefficient at a wavelength of 660 nm, and a large difference of about 100 times is provided. Meanwhile, since the absorption coefficient of light by deoxyhemoglobin indicated by a curved line of Hb becomes large at a wavelength of 700 nm or less, in order to eliminate this influence, the influence of absorption by deoxyhemoglobin may also be taken into consideration, and the measured value may be used to correct the value of the intravascular plasma volume. Such a configuration is effective in a case where an SpO$_2$ measuring function is also included in the intravascular plasma index measurement device 2. When performing measurement with high accuracy without being significantly affected by absorption by hemoglobin, it is preferable to use a wavelength of around 730 nm as indicated by a fourth dashed line 504. In this case, the difference between the first point 511 that exhibits the absorption coefficient at 970 nm and a fourth point 514 that exhibits the absorption coefficient at 730 nm is about 20 times. As indicated by a dashed line of an absorption curve of melanin (Melanin) in Fig. 8, the light absorption by melanin increases as the wavelength of a light source decreases. However, since melanin is mainly present in the skin and is not affected by pulsation, it affects both an alternating-current component and a direct-current component at any wavelength; therefore, as described in Step 308, by dividing the alternating-current component by the direct-current component, the influence thereof can be canceled out.

Next, in Step 406, the red light receiving unit 11 receives the second light transmitted through or reflected by the living body 6. Here, since the second light is less absorbed by water than the first light, the received second light undergoes less absorption by water due to blood pulsation than the first light, and a decrease in the light amount is smaller than that of the first light. On the other hand, the influence of absorption by melanin and the like on the second light is greater than that on the first light, and the light amount of the second light is reduced as compared with that of the first light.

Next, in Step 407, the red light emitting unit 32 turns off the second light, and the red light receiving unit 11 receives a second external light that is an external light from inside and outside the room and constitutes noise in a signal. The second external light is a light different from the second light, and is a light that does not include the second light emitted from the red light emitting unit 32. The second external light is, for example, an external light that the red light receiving unit 11 receives when the red light emitting unit 32 does not emit the second light.

Next, in Step 408, second photoelectric data obtained by subtracting a value of the photoelectric conversion of the second external light received in Step 407 from a value of the photoelectric conversion of the second light in Step 406 is calculated and accumulated. The steps from Step 405 to Step 408 are preferably performed at a rate of about 130 Hz as a sampling rate so as to avoid integer multiples of 50 Hz and 60 Hz of fluorescent lamps and the like that serve as external light.

Next, in Step 409, similarly to Step 305, the intravascular plasma index calculation circuit 17 determines whether or not the second photoelectric data on pulsation for a plurality of beats has been acquired. When the acquisition of the second photoelectric data is insufficient, the process returns to Step 401, and when the data acquisition is sufficient, the process proceeds to Step 410.

Next, in Step 410, similarly to Step 306, the intravascular plasma index calculation circuit 17 calculates the direct-current value $D_1$ from the first photoelectric data based on the intensity of the first light. The direct-current value $D_1$ takes the value as illustrated in Fig. 6(a).

Next, in Step 411, similarly to Step 307, the intravascular plasma index calculation circuit 17 calculates the alternating-current value $A_1$ that indicates the amplitude of the first light from the first photoelectric data. The alternating-current value $A_1$ may be an average value of the amplitude of a plurality of pulses. The alternating-current value $A_1$ may be, for example, a root mean square (RMS) value of the first photoelectric data, and takes the value as illustrated in Fig. 6(a).

Next, in Step 412, similarly to Step 306, the intravascular plasma index calculation circuit 17 calculates a direct-current value $D_2$ from the second photoelectric data based on the intensity of the second light. The direct-current value $D_2$ is a value that is less affected by absorption by water contained in plasma than the direct-current value $D_1$.

Next, in Step 413, similarly to Step 307, the intravascular plasma index calculation circuit 17 calculates an alternating-current value $A_2$ that indicates the amplitude of the second light from the second photoelectric data. The alternating-current value $A_2$ is a value that is less affected by absorption by water contained in plasma than the alternating-current value $A_1$. Fig. 6(b) is a diagram illustrating the direct-current value $D_2$ and the alternating-current value $A_2$ in the second photoelectric data. Fig. 6(d) is a diagram illustrating the direct-current value $D_2$ and the alternating-current value $A_2$ in the second photoelectric data in a state of water deficiency. The values are not subject to absorption by water as compared with the alternating-current value $A_1$ and the direct-current value $D_1$ of the first light. Since the alternating-current value $A_2$ and the direct-current value $D_2$ are less affected by water, they are not significantly affected even when plasma in blood vessels decreases, and thus, as illustrated in Fig. 6(d), no significant difference from Fig. 6(b) occurs. Further, even when melanin or the like in the skin changes due to suntan or the like and the waveform of Fig. 6(d) changes, the alternating-current value $A_2$ and the direct-current value $D_2$ are both affected by melanin, similarly to the relationship between the alternating-current value $A_1$ and the direct-current value $D_1$ described above in the explanation of Step 308, and therefore, by dividing the alternating-current value $A_2$ by the direct-current value $D_2$, it is possible to cancel out the influence caused by changes in melanin or the like as described below.

When a light having a wavelength of 660 nm, which is easily affected by hemoglobin, is emitted from the red light emitting unit 32 in Step 405, the alternating-current value $A_2$ and the direct-current value $D_2$ may be affected by hemoglobin. In particular, this influence is larger in the alternating-current value $A_2$ than in the direct-current value $D_2$. For example, when it is determined that the oxygen saturation is low, that is, that the proportion of oxyhemoglobin in the blood is small, there is a possibility that the proportion of deoxyhemoglobin having a high absorption coefficient is higher than that of oxyhemoglobin having a low light absorption coefficient at a wavelength of 660 nm. Therefore, the alternating-current value $A_2$ may be smaller than a normal value. In view of this, in Step 413, the $SpO_2$ measurement unit 23 may measure the oxygen information and correct the value of the alternating-current value $A_2$ based on the measured oxygen information. The $SpO_2$ measurement unit 23 may, for example, correct the value of the alternating-current value $A_2$ so as to increase in approximately inverse proportion to a rate of decrease in the blood oxygen saturation ($SpO_2$) indicated by the measured oxygen information.

Next, in Step 414, the intravascular plasma index calculation circuit 17 calculates $(A_1/D_1)/(A_2/D_2)$, and sets $(A_1/D_1)/(A_2/D_2)$ to the measurement value C. As illustrated in Fig. 6(c), the direct-current value $D_1$ decreases due to the influence of factors other than pulsation, and is attenuated and reduced at a rate common with that of the alternating-current value $A_1$. Therefore, by dividing the alternating-current value $A_1$ by the direct-current value $D_1$, common reduction factors are canceled out, resulting in a value having a high correlation with intravascular plasma. Further, since the alternating-current value $A_2$ is less susceptible to the influence of absorption by water than the alternating-current value $A_1$, the alternating-current value $A_2$ is also less susceptible to the influence of a decrease in the light amount due to a reduction in plasma. The direct-current value $D_2$ is also less susceptible to the influence of plasma than the direct-current value $D_1$. Therefore, changes in $(A_2/D_2)$ associated with increases and decreases in intravascular plasma are minor as compared with those in $(A_1/D_1)$.

Here, the alternating-current value $A_1$ measured with the first light having a large absorption coefficient of water is most susceptible to the influence of a reduction in intravascular plasma. Therefore, the measurement value C is calculated as a smaller value in association with the reduction in intravascular plasma. Depending on individual differences in the amount of capillaries, the strength of pulsation, and the like, there is a case where the amount of change in the alternating-current value $A_1$ becomes larger than the amount of change in the direct-current value $D_1$. However, in this case, the same applies to the alternating-current value $A_2$ and the direct-current value $D_2$, and the amount of change in the alternating-current value $A_2$ becomes larger than the amount of change in the direct-current value $D_2$. Accordingly, by dividing $(A_1/D_1)$ by $(A_2/D_2)$, it is possible to measure a stable numerical value without inter-individual variation of the living body 6.

Next, in Step 415, the intravascular plasma index calculation circuit 17 sets the average value $C_{AV}$. Here, similarly to Step 309, the intravascular plasma index calculation circuit 17 updates the measurement value C, that is, the average value of $(A_1/D_1)/(A_2/D_2)$ as the average value $C_{AV}$ by weighting and apportioning the average value of $(A_1/D_1)/(A_2/D_2)$ based on past usage time. When two different wavelengths of the first light and the second light are used, the inter-individual variation of the average value $C_{AV}$ of the living body 6 becomes very small compared with a case where only the first light is used. Therefore, when a plurality of living bodies 6 alternately use the intravascular plasma index measurement system 100, the intravascular plasma index calculation circuit 17 may set $C_{AV}$ as a constant in order to prioritize convenience. The intravascular plasma index calculation circuit 17 may, for example, preset the average value $C_{AV}$ = 1.35 at all times, and omit Step 415.

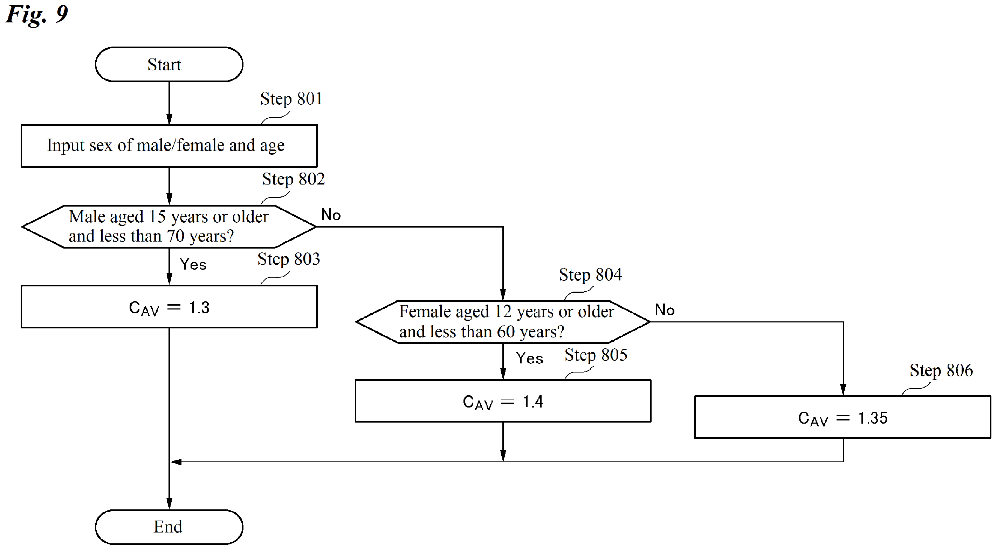

In comparison between adult males and adult females, adult females may have a higher proportion of plasma. This is considered to be because females have less hemoglobin in the blood and are closer to a so-called anemic condition than males. Further, the proportion of plasma differs depending on attributes of the living body 6, for example, age. Therefore, for example, in Step 415, attribute information related to the attributes of the living body 6 may be acquired, and the average value $C_{AV}$ may be set based on the acquired attribute information. The attribute information is, for example, information on the sex, age, race, nationality, medical history, and the like of the living body 6. Fig. 9 is a diagram illustrating an operation of setting the average value $C_{AV}$ by the intravascular plasma index measurement system 100 according to the embodiment. In this case, the intravascular plasma index calculation circuit 17 performs an operation according to the flowchart as illustrated in Fig. 9.

First, in Step 801, the intravascular plasma index calculation circuit 17 acquires the attribute information including information indicating whether the living body 6 as a measurement target is a male or a female and information on the age of the living body. The intravascular plasma index calculation circuit 17 may acquire the information by any method. The information on the age may be information on the current age or information on the date of birth.

Next, in Step 802, the intravascular plasma index calculation circuit 17 determines whether the living body 6 is a "male aged 15 years or older and less than 70 years" based on the information acquired in Step 801. When the living body 6 is a "male aged 15 years or older and less than 70 years," the intravascular plasma index calculation circuit 17 proceeds to Step 803. When the living body 6 is not a "male aged 15 years or older and less than 70 years" in Step 802, the intravascular plasma index calculation circuit 17 proceeds to Step 804. In Step 804, whether the living body 6 is a "female aged 12 years or older and less than 60 years" or not is determined. When the living body 6 is a "female aged 12 years or older and less than 60 years," the intravascular plasma index calculation circuit 17 proceeds to Step 805, and otherwise proceeds to Step 806.

In Step 803, the intravascular plasma index calculation circuit 17 sets the average value $C_{AV}$ = 1.3. In Step 805, the intravascular plasma index calculation circuit 17 sets the average value $C_{AV}$ = 1.4. In Step 806, the intravascular plasma index calculation circuit 17 sets the average value $C_{AV}$ = 1.35 because those aged 70 years or older or less than 15 years are targeted when the living body 6 is a male, those aged 60 years or less than 12 years are targeted when the living body 6 is a female, and there is almost no difference depending on sex. The average value $C_{AV}$ may be set at any timing. For example, the average value $C_{AV}$ may be preset before the use of the intravascular plasma index measurement system 100, or may be set in Step 415. The value of the average value $C_{AV}$ may be set more finely according to age, for example, in increments of 0.01.

For example, the housings 300 for males, females, children, and elderly persons may be prepared in advance, and the average value $C_{AV} = 1.35$ may be set when the housing 300 for males is used, the average value $C_{AV} = 1.4$ may be set when the housing 300 for females is used, and the average value $C_{AV} = 1.35$ may be set when the housings 300 for children and elderly persons are used.

Next, in Step 416, similarly to Step 310, the intravascular plasma index calculation circuit 17 calculates the intravascular plasma index M. In Step 416, the intravascular plasma index calculation circuit 17 calculates the intravascular plasma index M by raising the value of C - $C_{AV}$ to the n-th power, further multiplying the resulting value by the constant K, and adding the constant L, as shown in, for example, an equation (5) and an equation (6).

$$C = (A_1/D_1)/(A_2/D_2) \quad (5)$$

$$M = K(C - C_{AV})^n + L \quad (6)$$

Here, K, $C_{AV}$, L, and *n* are constants.

In Step 416, the intravascular plasma index calculation circuit 17 calculates the intravascular plasma index M by raising the value of C - $C_{AV}$ to the n-th power, further multiplying the resulting value by the constant K, and adding the constant L, as shown in, for example, an equation (7) and an equation (8).

$$C = (A_1/D_1)/(A_2/D_2) \quad (7)$$

$$M = K(C - C_{AV})^n + L \quad (8)$$

Here, K, L, and *n* are constants, and $C_{AV}$ is an average value of $(A_1/D_1)/(A_2/D_2)$.

In Step 416, the intravascular plasma index calculation circuit 17 may calculate the intravascular plasma index M, for example, using an equation (9) and an equation (10).

$$C = (A_1/D_1)/(A_2/D_2) \quad (9)$$

$$M = f(C - C_{AV}) \quad (10)$$

Here, f(x) is a function in which a differential value obtained by differentiating f(x) with respect to *x* when *x* is equal to or greater than the constant g, that is, a slope is smaller than a differential value when x is smaller than the constant g. The function f(x) may be, for example, a linear function or a function of second or higher order in which the slope becomes smaller when x exceeds g.

Thus, the operation of measuring the intravascular plasma index M using the first light and the second light is completed. Accordingly, the intravascular plasma index M can be calculated with high accuracy using $(A_1/D_1)/(A_2/D_2)$ having a high correlation with intravascular plasma from which noise has been removed. Compared with a case where the intravascular plasma index M is measured using only the first light, the influence of individual differences of the living body 6 is reduced, and therefore, even when the plurality of living bodies 6 use the same intravascular plasma index measurement device 2, the influence is small.

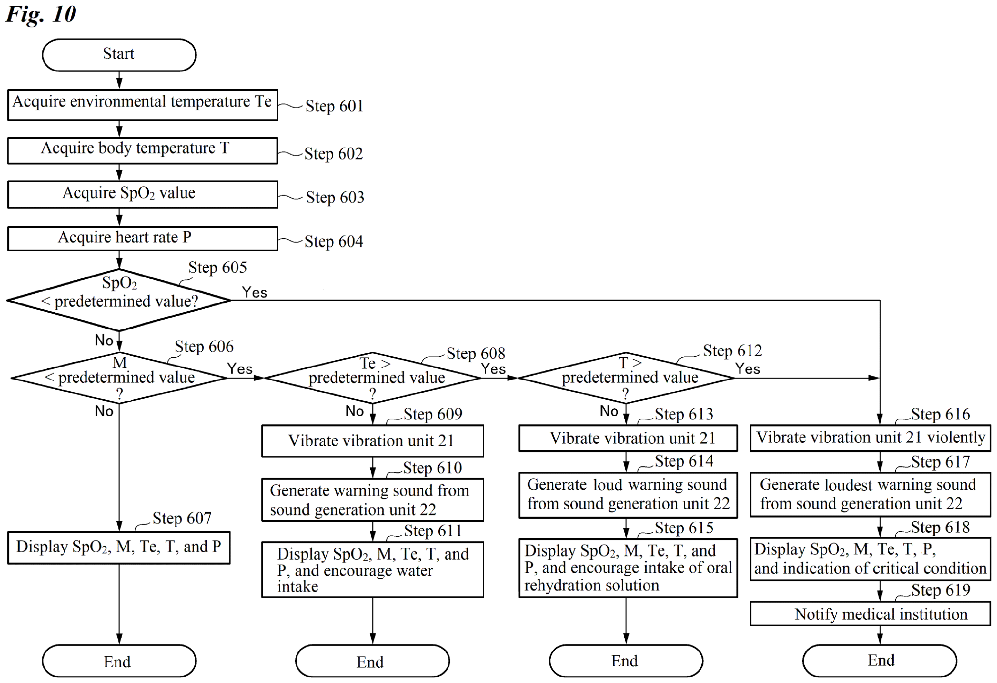

Next, an exemplary operation after the calculation of the intravascular plasma index M in the intravascular plasma index measurement system 100 according to the embodiment will be described. Fig. 10 is a diagram illustrating a flowchart of the operation after the calculation of the intravascular plasma index M in the intravascular plasma index measurement system 100 according to the embodiment. After calculating the intravascular plasma index M in Step 310 or Step 416, the intravascular plasma index calculation circuit 17 proceeds to Step 601.

First, in Step 601, the environmental temperature measurement unit 33a acquires environmental temperature information Te.

Next, in Step 602, the temperature difference measurement unit 33b measures a temperature difference between the measurement site of the living body 6 (skin or the like of the living body) and the environment, and adds the temperature difference to the environmental temperature information measured in Step 601, thereby calculating the temperature information including the body temperature T of the living body 6. The temperature information is information related to temperatures, and includes, for example, the body temperature T of the living body, the temperature difference information, and the environmental temperature information.

Next, in Step 603, the $SpO_2$ measurement unit 23 acquires the oxygen information. In this case, when the measurement is performed using a known method of acquiring the oxygen information with infrared light, by setting the wavelength of the infrared light to 1000 nm or less, it becomes possible to reduce the size of the light receiving element, and thus, the overall size of the intravascular plasma index measurement device 2 can be reduced.

Next, in Step 604, the intravascular plasma index calculation circuit 17 acquires the pulse information related to the pulse rate P of the living body 6. The intravascular plasma index calculation circuit 17, for example, may calculate the heart rate P for one minute based on the photoelectric data acquired in Step 409 described above, and use the calculated heart rate P as the pulse information.

Next, in Step 605, the control circuit 18 determines whether the living body 6 has an abnormality or not based on the oxygen information. For example, when the value of $SpO_2$ indicated by the oxygen information is equal to or less than a predetermined value, that is, when the blood oxygen saturation decreases and it is determined that the living body 6 is in danger, the control circuit 18 proceeds to Step 616 and performs an emergency response. For example, when the value of $SpO_2$ indicated by the oxygen information is larger than the predetermined value, the control circuit 18 proceeds to Step 606. The abnormality of the living body 6 is, for example, heatstroke or dehydration, but is not limited thereto, and may be a tendency to any symptom.

Next, in Step 606, the control circuit 18 determines whether the living body 6 has an abnormality or not based on the intravascular plasma index M. In Step 606, the control circuit 18 determines, for example, whether the intravascular plasma index M is smaller than a predetermined value or not. When the intravascular plasma index M is smaller than the predetermined value, the control circuit 18 determines that water in blood vessels is insufficient and proceeds to Step 608. When the intravascular plasma index M is equal to or more than the predetermined value, the control circuit 18 proceeds to Step 607.

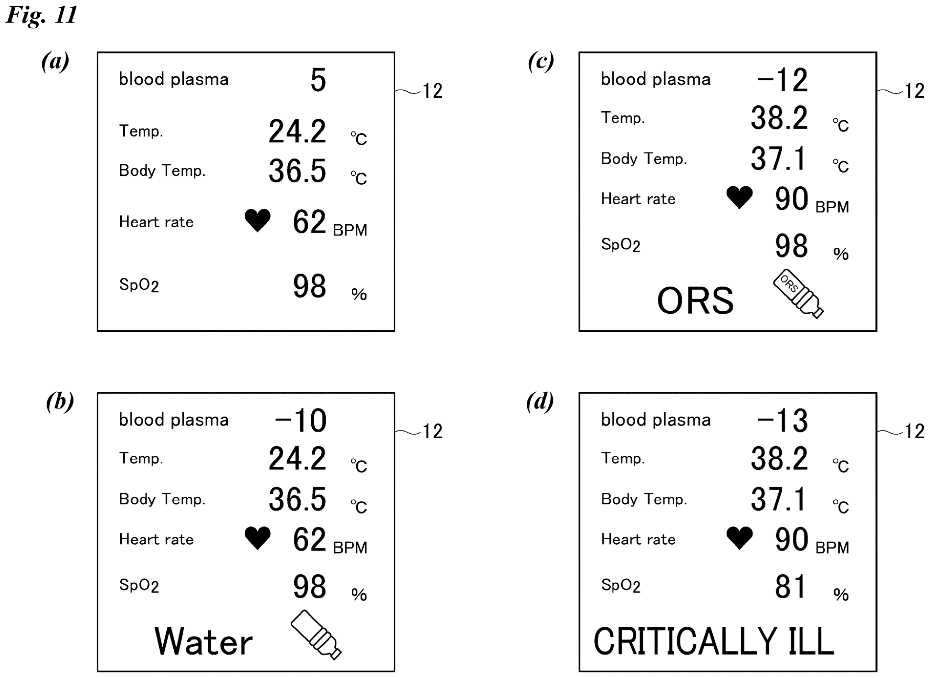

Fig. 11 is diagrams illustrating exemplary display units 12. Next, in Step 607, the display unit 12 displays, for example, as illustrated in Fig. 11(a), the intravascular plasma index (blood plasma) M, the environmental temperature (Temp.) Te, the body temperature (Body Temp.) T, the heart rate (Heart rate) P, and the blood oxygen saturation ($SpO_2$) $SpO_2$ value. The intravascular plasma index measurement system 100 may proceed to Step 301, Step 401, or Step 601 again.

Next, in Step 608, the control circuit 18 determines whether the living body 6 has an abnormality or not based on the temperature information. In Step 608, the control circuit 18 determines, for example, whether the environmental temperature Te is higher than a predetermined value or not. When the environmental temperature Te is higher than the predetermined value, since the intravascular plasma index M of the living body is low, there is a possibility of dehydration, and the environmental temperature is high, the control circuit 18 determines that the living body 6 is in danger and proceeds to Step 612. When the environmental temperature Te is equal to or less than the predetermined value, the control circuit 18 determines that a rapid decrease in water in plasma due to perspiration or the like is unlikely to occur and that there is no serious danger, and proceeds to Step 609. As dehydration progresses, the heart rate increases by approximately 4 to 6 bpm for each 1% decrease in body weight. While the heart rate also increases due to exercise or the like, when there is a tendency toward dehydration and the heart rate P acquired in Step 604 is higher than normal, the risk of dehydration is high, and therefore, the process may proceed to Step 602, and a significant warning may be issued.

Next, in Step 609, the vibration unit 21 generates vibration to issue a warning to the living body 6. Accordingly, even when the living body 6 focuses on an event or the like, it is possible to inform the living body 6 of the possibility of dehydration and the danger of neglecting a water intake.

Next, in Step 610, the sound generation unit 22 generates a sound or a voice in order to notify of a danger. This makes it possible to notify the living body 6 and surrounding third parties of the danger.

Next, in Step 611, the display unit 12 displays, for example, as illustrated in Fig. 11(b), the intravascular plasma index M, the environmental temperature Te, the body temperature T, the heart rate P, and the $SpO_2$ value, and further displays an indication for a water intake, thereby encouraging water intake. Here, when water replenishment is required for the living body 6, the display unit 12 may display, as illustrated in FIG. 11(b), a plastic bottle together with an indication of water (Water). The intravascular plasma index measurement system 100 may proceed to Step 301, Step 401, or Step 601 again.

When a large amount of water is ingested, for example, in response to thirst, the proportion of plasma in the blood temporarily increases. This increase in the proportion of plasma in the blood normally does not cause a problem, and the proportion is known to return to a normal value over time, but in some cases, the increase may adversely affect the living body 6. For example, when the proportion of plasma in the blood increases, the amount of water, which is a main component thereof, increases, thereby increasing intraocular pressure. Such increased intraocular pressure causes, for example, the onset of glaucoma. In this regard, for a person who usually has a relatively high intraocular pressure, when the proportion of plasma is large, the display unit 12 may issue a warning, display an indication of refraining from a further water intake, and encourage the person to slowly take in water after the warning is cleared.

Next, in Step 612, the control circuit 18 determines whether the body temperature T is higher than a predetermined value or not. When the body temperature T is higher than the predetermined value, there is a possibility that body temperature regulation by perspiration is not functioning properly, and since the environmental temperature Te is also high, the control circuit 18 determines that the living body 6 is in danger and proceeds to Step 616. When the body temperature T is equal to or less than the predetermined value, the control circuit 18 proceeds to Step 613.

Next, in Step 613, since the intravascular plasma index of the living body is low and the environmental temperature is high, the living body is in a state where the risk of dehydration is higher than in the state of Step 609. Therefore, the vibration unit 21 generates vibration to issue a warning to the living body 6. Accordingly, even when the living body 6 focuses on an event or the like, it is possible to inform the living body 6 of a danger.

Next, in Step 614, the sound generation unit 22 generates a sound or a voice in order to notify of a danger. In Step 614, the sound generation unit 22 may generate a sound or a voice louder than that in Step 610. This makes it possible to notify the living body 6 and surrounding third parties of the danger.

Next, in Step 615, the display unit 12 displays, as illustrated in Fig. 11(c), the intravascular plasma index M, the environmental temperature Te, the body temperature T, and the $SpO_2$ value, and since the state is more urgent, displays an indication of obtaining an oral rehydration solution supplemented with various minerals rather than simple water, thereby encouraging an intake of necessary water and minerals. When supplementation of an oral rehydration solution is required for the living body 6, the display unit 12 may display, as illustrated in FIG. 11(c), a plastic bottle of an oral rehydration solution together with an indication of an oral rehydration solution (ORS). The intravascular plasma index measurement system 100 may proceed to Step 301, Step 401, or Step 601 again.

Next, in Step 616, since the intravascular plasma index M is low, the environmental temperature Te and the body temperature T are high, and the living body is in an extremely dangerous condition, the vibration unit 21 generates strong vibrations to issue a warning to the living body 6. Accordingly, even in a situation where thinking of the living body 6 is partially impaired, it is possible to inform the living body 6 of a serious danger.

Next, in Step 617, the sound generation unit 22 generates a sound or a voice in order to notify of a danger. In Step 617, the sound generation unit 22 may generate a sound or a voice louder than that in Step 614. This makes it possible to notify the living body 6 and surrounding third parties of a serious danger.

Next, in Step 618, the display unit 12 displays, as illustrated in Fig. 11(d), $SpO_2$, the intravascular plasma index M, the environmental temperature Te, the body temperature T, and the heart rate P, and displays an indication that the condition is severe. When it is determined that the living body 6 is in a life-threatening condition, the display unit 12 may display an indication of critical condition (CRITICALLY ILL) as illustrated in Fig. 11(d).

Next, in Step 619, the communication unit 13 notifies a medical institution and prompts action to ensure the safety of the living body 6. The intravascular plasma index measurement system 100 may proceed to Step 301, Step 401, or Step 601 again.

While the embodiment of the present invention has been described, the embodiment has been presented as an example, and is not intended to limit the scope of the invention. This novel embodiment can be embodied in a variety of other configurations. Various omissions, substitutions, and changes can be made without departing from the gist of the invention. The embodiment and the modifications thereof are within the scope and the gist of the invention and within the scope of the invention described in the claims and its equivalents.

DESCRIPTION OF REFERENCE SIGNS

| 1: | Sensor |
|---|---|
| 2: | Intravascular plasma index measurement device |
| 3: | Server |
| 4: | Public communications network |
| 6: | Living body |
| 7: | User terminal |
| 10: | Infrared light receiving unit |
| 11: | Red light receiving unit |
| 12: | Display unit |
| 13: | Communication unit |
| 14: | Connector |
| 15: | Power switch |
| 17: | Intravascular plasma index calculation circuit |
| 18: | Control circuit |
| 19: | Battery |
| 21: | Vibration unit |
| 22: | Sound generation unit |
| 23: | $SpO_2$ measurement unit |
| 26: | Contact sensor |
| 31: | Infrared light emitting unit |
| 32: | Red light emitting unit |
| 33: | Temperature measurement unit |
| 33a: | Environmental temperature measurement unit |
| 33b: | Temperature difference measurement unit |
| 36: | Light emission control circuit |
| 37: | Body temperature calculation circuit |
| 41: | First housing |
| 100: | Intravascular plasma index measurement system |
| 300: | Housing |
| 301: | Upper housing |
| 302: | Upper recess |
| 304: | Shaft |
| 341: | Lower housing |
| 342: | Lower recess |
| 400: | Housing |
| 401a: | Front surface |
| 401b: | Back surface |
| 402: | Wristband |
| 501: | First dashed line |
| 502: | Second dashed line |
| 503: | Third dashed line |
| 504: | Fourth dashed line |
| 511: | First point |
| 512: | Second point |
| 513: | Third point |

514:      Fourth point
550:      First region
551:      Second region
552:      Third region

**Claims**

1. An intravascular plasma index measurement system comprising:

   first irradiation means that irradiates a living body with a first light;
   first light receiving means that receives the first light emitted from the first irradiation means and transmitted through or reflected by the living body;
   first calculation means that calculates a measurement value C related to an amplitude of the first light for $T_1$ seconds longer than a heartbeat cycle of the living body based on the first light received by the first light receiving means;
   second calculation means that calculates an average value $C_{AV}$ related to the amplitude of the first light for $T_2$ seconds longer than the $T_1$ seconds based on the first light received by the first light receiving means; and
   third calculation means that calculates an intravascular plasma index M related to intravascular plasma of the living body based on the measurement value C calculated by the first calculation means and the average value $C_{AV}$ calculated by the second calculation means.

2. The intravascular plasma index measurement system according to claim 1, wherein

   the third calculation means calculates the intravascular plasma index M using an equation (1) below:

   $$M = K(C - C_{AV})^n + L \quad (1)$$

   wherein K, L, and $n$ are constants.

3. The intravascular plasma index measurement system according to claim 1, wherein

   the third calculation means calculates the intravascular plasma index M using an equation (2) below:

   $$M = f(C - C_{AV}) \quad (2)$$

   wherein f(x) is a function in which a differential value obtained by differentiating f(x) with respect to $x$ when $x$ is equal to or greater than a constant $g$ is smaller than a differential value when $x$ is smaller than the constant $g$.

4. The intravascular plasma index measurement system according to claim 1, wherein

   the first calculation means extracts an alternating-current value $A_1$ that indicates the amplitude of the first light and a direct-current value $D_1$ based on an intensity of the first light based on the first light received by the first light receiving means, and calculates the measurement value C based on the extracted alternating-current value $A_1$ and the extracted direct-current value $D_1$.

5. The intravascular plasma index measurement system according to claim 4, wherein

   the third calculation means calculates the intravascular plasma index M using an equation (3) below:

   $$M = K(C - C_{AV})^n + L \quad (3)$$

   wherein K, L, and $n$ are constants, and $C = A_1/D_1$ is defined.

6. The intravascular plasma index measurement system according to claim 4, wherein

   the third calculation means calculates the intravascular plasma index M using an equation (4) below:

   $$M = f(C - C_{AV}) \quad (4)$$

   wherein f(x) is a function in which a differential value obtained by differentiating f(x) with respect to $x$ when $x$ is equal to or greater than a constant g is smaller than a differential value when x is smaller than the constant g, and $C = A_1/D_1$ is defined.

7. An intravascular plasma index measurement system comprising:

   first irradiation means that irradiates a living body with a first light;
   first light receiving means that receives the first light emitted from the first irradiation means and transmitted through or reflected by the living body;
   first calculation means that extracts an alternating-current value $A_1$ that indicates an amplitude of the first light and a direct-current value $D_1$ based on an intensity of the first light based on the first light received by the first light receiving means;
   second irradiation means that irradiates the living body with a second light having a wavelength that is shorter than a wavelength of the first light and at which an absorption characteristic of water is lower than at the wavelength of the first light;
second light receiving means that receives the second light emitted from the second irradiation means and transmitted through or reflected by the living body;
second calculation means that extracts an alternating-current value $A_2$ that indicates an amplitude of the second light and a direct-current value $D_2$ based on an intensity of the second light based on the second light received by the second light receiving means; and
third calculation means that calculates an intravascular plasma index M related to intravascular plasma of the living body based on the alternating-current value $A_1$, the alternating-current value $A_2$, the direct-current value $D_1$, and the direct-current value $D_2$ calculated by the first calculation means and the second calculation means.

8. The intravascular plasma index measurement system according to claim 7, wherein

the third calculation means calculates the intravascular plasma index M using an equation (5) and an equation (6) below:

$$C = (A_1/D_1)/(A_2/D_2) \quad (5)$$

$$M = K(C - C_{AV})^n + L \quad (6)$$

wherein K, $C_{AV}$, L, and $n$ are constants.

9. The intravascular plasma index measurement system according to claim 7, wherein

the third calculation means calculates the intravascular plasma index M using an equation (7) and an equation (8) below:

$$C = (A_1/D_1)/(A_2/D_2) \quad (7)$$

$$M = K(C - C_{AV})^n + L \quad (8)$$

wherein K, L, and $n$ are constants, and $C_{AV}$ is an average value of $(A_1/D_1)/(A_2/D_2)$.

10. The intravascular plasma index measurement system according to claim 7, wherein

the third calculation means calculates the intravascular plasma index M using an equation (9) and an equation (10) below:

$$C = (A_1/D_1)/(A_2/D_2) \quad (9)$$

$$M = f(C - C_{AV}) \quad (10)$$

wherein f(x) is a function in which a differential value obtained by differentiating f(x) with respect to $x$ when $x$ is equal to or greater than a constant g is smaller than a differential value when x is smaller than the constant g, and $C_{AV}$ is an average value of $(A_1/D_1)/(A_2/D_2)$ or a constant.

11. The intravascular plasma index measurement system according to claim 1, wherein
the first light has a wavelength within any range of 960 nm to 980 nm, 1440 nm to 1460 nm, and 1930 nm to 1950 nm.

12. The intravascular plasma index measurement system according to claim 7, wherein

the first light has a wavelength within any range of 960 nm to 980 nm, 1440 nm to 1460 nm, and 1930 nm to 1950 nm, and
the second light has a wavelength of 800 nm or less.

13. The intravascular plasma index measurement system according to claim 1, further comprising any one or more of:

display means that displays the intravascular plasma index M calculated by the third calculation means;
vibration means that generates a vibration based on the intravascular plasma index M calculated by the third calculation means;
communication means that transmits the intravascular plasma index M calculated by the third calculation means to an external device; and
sound means that generates a sound based on the intravascular plasma index M calculated by the third calculation means.

14. The intravascular plasma index measurement system according to claim 1, further comprising:

acquisition means that acquires temperature information related to a temperature; and
determination means that determines an abnormality of the living body based on the temperature information acquired by the acquisition means and the intravascular plasma index M calculated by the third calculation means.

15. The intravascular plasma index measurement system according to claim 1, further comprising:

acquisition means that acquires oxygen information related to blood oxygen saturation of the living body; and

determination means that determines an abnormality of the living body based on the oxygen information acquired by the acquisition means and the intravascular plasma index M calculated by the third calculation means.

16. The intravascular plasma index measurement system according to claim 1, wherein

the first light receiving means receives a first external light different from the first light, and the first calculation means corrects the measurement value C based on the first external light received by the first light receiving means.

17. The intravascular plasma index measurement system according to claim 7, wherein

the first light receiving means receives a first external light different from the first light,

the first calculation means corrects any one or more of the alternating-current value $A_1$ and the direct-current value $D_1$ based on the first external light received by the first light receiving means,

the second light receiving means receives a second external light different from the second light, and

the second calculation means corrects any one or more of the alternating-current value $A_2$ and the direct-current value $D_2$ based on the second external light received by the second light receiving means.

18. The intravascular plasma index measurement system according to claim 1, further comprising:

acquisition means that acquires pulse information related to a pulse rate of the living body; and

determination means that determines an abnormality of the living body based on the pulse information acquired by the acquisition means and the intravascular plasma index M calculated by the third calculation means.

19. The intravascular plasma index measurement system according to claim 1, further comprising:

acquisition means that acquires any one or more of temperature information related to a temperature, oxygen information related to blood oxygen saturation of the living body, and pulse information related to a pulse rate of the living body; and

determination means that determines a type of water prescribed to the living body based on any one or more of the temperature information, the oxygen information, and the pulse information acquired by the acquisition means, and the intravascular plasma index M calculated by the third calculation means.

20. The intravascular plasma index measurement system according to claim 8, wherein

the third calculation means acquires attribute information related to an attribute of the living body, and sets the $C_{AV}$ based on the acquired attribute information.

*Fig. 1*

*Fig. 2*

1

300

301

302

31,32

342

341

304

10,11

**Fig. 3**

*(a)*

| | |
|---|---|
| blood plasma | 11 |
| Temp. | 32.2 |
| Body Temp. | 27.5 |
| Heart rate ♥ | 62 |

*(b)*

EP 4 763 079 A1

**Fig. 4**

First Housing

| | |
|---|---|
| Display Unit — 12 | |
| Communication Unit — 13 | Vibration Unit — 21 |
| Connector — 14 | Sound Generation Unit — 22 |
| Power Switch — 15 | SpO₂ Measurement Unit — 23 |

Infrared Light Receiving Unit — 10

Red Light Receiving Unit — 11

Intravascular Plasma Index Calculation Circuit — 17

Control Circuit — 18

Battery — 19

Infrared Light Emitting Unit — 31

Red Light Emitting Unit — 32

Light Emission Control Circuit — 36

Contact Sensor — 26

Environmental Temperature Measurement Unit — 33a

Temperature Difference Measurement Unit — 33b

Body Temperature Calculation Circuit — 37

33

1

6

2

41

EP 4 763 079 A1

26

## Fig. 5

**(a)**

```
                    ( Start )
                        │
                        ▼                          ⌐Step 301
┌──────────────────────────────────────────────┐
│  Emit first light from infrared light emitting unit 31  │
└──────────────────────────────────────────────┘
                        │                          ⌐Step 302
                        ▼
┌──────────────────────────────────────────────┐
│  Receive transmitted or reflected first light by infrared
│  light receiving unit 10                       │
└──────────────────────────────────────────────┘
                        │                          ⌐Step 303
                        ▼
┌──────────────────────────────────────────────┐
│  Turn off infrared light emitting unit 31 and receive
│  background noise                              │
└──────────────────────────────────────────────┘
                        │                          ⌐Step 304
                        ▼
┌──────────────────────────────────────────────┐
│  Subtract signal at turn-off from signal at irradiation,
│  and accumulate first photoelectric data       │
└──────────────────────────────────────────────┘
                        │
                        ▼                          ⌐Step 305
 No       ◇ Data on pulsation for a plurality of beats acquired? ◇
                        │ Yes
                        ▼                          ⌐Step 306
┌──────────────────────────────────────────────┐
│  Calculate direct-current value D1 of direct-current
│  (DC) component from first photoelectric data  │
└──────────────────────────────────────────────┘
                        │                          ⌐Step 307
                        ▼
┌──────────────────────────────────────────────┐
│  Calculate alternating-current value A1 of alternating-
│  current (AC) component from first photoelectric data  │
└──────────────────────────────────────────────┘
                        │                          ⌐Step 308
                        ▼
┌──────────────────────────────────────────────┐
│               Calculate A1/D1                  │
└──────────────────────────────────────────────┘
                        │                          ⌐Step 309
                        ▼
┌──────────────────────────────────────────────┐
│  Correct average value $C_{AV}$ corresponding to rate of
│  the number of past measurements using additionally
│  measured (A1/D1) value                        │
└──────────────────────────────────────────────┘
                        │                          ⌐Step 310
                        ▼
┌──────────────────────────────────────────────┐
│  Calculate intravascular plasma index M        │
│  M = K•(C − $C_{AV}$)ⁿ + L (note that  C = A1/D1)  │
└──────────────────────────────────────────────┘
                        │
                        ▼
                    ( End )
```

Step 310

$$M = K \cdot (C - C_{AV})^n + L \quad (\text{note that } C = A1/D1)$$

## Fig. 5
**(b)**

```
                           ( Start )
```

| | |
|---|---|
| Emit first light from infrared light emitting unit 31 | ∼Step 401 |
| Receive transmitted or reflected first light by infrared light receiving unit 10 | ∼Step 402 |
| Turn off infrared light emitting unit 31 and receive background noise | ∼Step 403 |
| Subtract signal at turn-off from signal at irradiation, and accumulate first photoelectric data | ∼Step 404 |
| Emit second light from red light emitting unit 32 | ∼Step 405 |
| Receive transmitted or reflected second light by red light receiving unit 11 | ∼Step 406 |
| Turn off red light emitting unit 32 and receive background noise | ∼Step 407 |
| Subtract signal at turn-off from signal at irradiation, and accumulate second photoelectric data | ∼Step 408 |

No ◁ Data on pulsation for a plurality of beats acquired? ▷ ⟋Step 409

↓ Yes

| | |
|---|---|
| Calculate direct-current value D1 of direct-current (DC) component from first photoelectric data | ∼Step 410 |
| Calculate alternating-current value A1 of alternating-current (AC) component from first photoelectric data | ∼Step 411 |
| Calculate direct-current value D2 of direct-current (DC) component from second photoelectric data | ∼Step 412 |
| Calculate alternating-current value A2 of alternating-current (AC) component from second photoelectric data | ∼Step 413 |
| Calculate (A1/D1)/(A2/D2) | ∼Step 414 |
| Correct average value $C_{AV}$ corresponding to rate of the number of past measurements using additionally measured (A1/D1)/(A2/D2) value | ∼Step 415 |
| Calculate intravascular plasma index M $M = K \cdot (C - C_{AV})^n + L$, note that $C = ((A1/D1)/(A2/D2))$ | ∼Step 416 |

```
                           ( End )
```

**Fig. 6**

*(a)*

*(b)*

*(c)*

*(d)*

Fig. 7

M = f (C–Cav)

EP 4 763 079 A1

*Fig. 8*

## Fig. 9

```
                    ┌─────────────┐
                    │    Start    │
                    └──────┬──────┘
                           │        Step 801
                    ┌──────▼──────────────────┐
                    │ Input sex of male/female │
                    │         and age          │
                    └──────┬──────────────────┘
                           │        Step 802
                  ┌────────▼─────────────┐   No
                 ╱ Male aged 15 years or  ╲──────────┐
                ╱  older and less than      ╲         │
                ╲  70 years?                ╱         │
                 ╲────────┬─────────────┘            │
                   Yes    │   Step 803                │
                ┌─────────▼──────────┐                │
                │                    │                │
                │   C_AV = 1.3       │                │
                └─────────┬──────────┘                ▼     Step 804
                          │              ┌───────────────────────┐   No
                          │             ╱ Female aged 12 years or  ╲──────────┐
                          │            ╱  older and less than        ╲         │
                          │            ╲  60 years?                   ╱         │
                          │             ╲──────────┬─────────────┘            │
                          │               Yes      │   Step 805                │
                          │            ┌───────────▼──────────┐                ▼   Step 806
                          │            │     C_AV = 1.4        │     ┌───────────────────┐
                          │            └───────────┬──────────┘     │   C_AV = 1.35     │
                          │                        │                └─────────┬─────────┘
                          │◄───────────────────────┴──────────────────────────┘
                          ▼
                    ┌─────────────┐
                    │     End     │
                    └─────────────┘
```

Step 801 — Input sex of male/female and age

Step 802 — Male aged 15 years or older and less than 70 years?

Step 803 — $C_{AV} = 1.3$

Step 804 — Female aged 12 years or older and less than 60 years?

Step 805 — $C_{AV} = 1.4$

Step 806 — $C_{AV} = 1.35$

EP 4 763 079 A1

**Fig. 10**

Start

Acquire environmental temperature Te — Step 601

Acquire body temperature T — Step 602

Acquire SpO$_2$ value — Step 603

Acquire heart rate P — Step 604

Step 605
SpO$_2$ < predetermined value? — Yes

No

Step 606
M < predetermined value? — Yes

Step 608
Te > predetermined value? — Yes

Step 612
T > predetermined value? — Yes

No

No — Step 609
Vibrate vibration unit 21

No — Step 613
Vibrate vibration unit 21

Step 616
Vibrate vibration unit 21 violently

Step 610
Generate warning sound from sound generation unit 22

Step 614
Generate loud warning sound from sound generation unit 22

Step 617
Generate loudest warning sound from sound generation unit 22

Step 607
Display SpO$_2$, M, Te, T, and P

Step 611
Display SpO$_2$, M, Te, T, and P, and encourage water intake

Step 615
Display SpO$_2$, M, Te, T, and P, and encourage intake of oral rehydration solution

Step 618
Display SpO$_2$, M, Te, T, P, and indication of critical condition

Step 619
Notify medical institution

End

End

End

End

EP 4 763 079 A1

**Fig. 11**

(a)

| | | |
|---|---|---|
| blood plasma | 5 | |
| Temp. | 24.2 | ℃ |
| Body Temp. | 36.5 | ℃ |
| Heart rate | ♥ 62 | BPM |
| SpO₂ | 98 | % |

~12

(b)

| | | |
|---|---|---|
| blood plasma | −10 | |
| Temp. | 24.2 | ℃ |
| Body Temp. | 36.5 | ℃ |
| Heart rate | ♥ 62 | BPM |
| SpO₂ | 98 | % |

Water

~12

(c)

| | | |
|---|---|---|
| blood plasma | −12 | |
| Temp. | 38.2 | ℃ |
| Body Temp. | 37.1 | ℃ |
| Heart rate | ♥ 90 | BPM |
| SpO₂ | 98 | % |

ORS

~12

(d)

| | | |
|---|---|---|
| blood plasma | −13 | |
| Temp. | 38.2 | ℃ |
| Body Temp. | 37.1 | ℃ |
| Heart rate | ♥ 90 | BPM |
| SpO₂ | 81 | % |

CRITICALLY ILL

~12

## INTERNATIONAL SEARCH REPORT

| International application No. |
| --- |
| **PCT/JP2023/046667** |

| A. | CLASSIFICATION OF SUBJECT MATTER |
| --- | --- |

*A61B 5/1455*(2006.01)i
FI:   A61B5/1455

According to International Patent Classification (IPC) or to both national classification and IPC

| B. | FIELDS SEARCHED |
| --- | --- |

Minimum documentation searched (classification system followed by classification symbols)

A61B5/145-5/1455; A61B5/00-5/03; A61B10/00; G01N21/17; G01N21/27; G01N21/35; G01N33/483

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | WO 2004/006769 A1 (SEIKO EPSON CORPORATION) 22 January 2004 (2004-01-22) p. 7, line 16 to p. 15, line 14, fig. 1-7 | 1-20 |
| A | JP 2004-81427 A (YOSHIKAWA, Kenji) 18 March 2004 (2004-03-18) paragraphs [0010]-[0060], fig. 1-7 | 1-20 |
| A | JP 2018-153357 A (SHARP KABUSHIKI KAISHA) 04 October 2018 (2018-10-04) paragraphs [0026]-[0135], fig. 1-26 | 1-20 |
| A | JP 2017-518792 A (KONINKLIJKE PHILIPS N.V.) 13 July 2017 (2017-07-13) paragraphs [0024]-[0039], fig. 1-2 | 1-20 |

☐ Further documents are listed in the continuation of Box C.       ☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **11 March 2024** | **26 March 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

**INTERNATIONAL SEARCH REPORT**
Information on patent family members

International application No.

**PCT/JP2023/046667**

| Patent document cited in search report | | | Publication date (day/month/year) | Patent family member(s) | | | Publication date (day/month/year) |
|---|---|---|---|---|---|---|---|
| WO | 2004/006769 | A1 | 22 January 2004 | US 2004/0210144 A1 paragraphs [0058]-[0097], fig. 1-7 CN 1662178 A | | | |
| JP | 2004-81427 | A | 18 March 2004 | (Family: none) | | | |
| JP | 2018-153357 | A | 04 October 2018 | (Family: none) | | | |
| JP | 2017-518792 | A | 13 July 2017 | US 2017/0202493 A1 paragraphs [0032]-[0047], fig. 1-2 WO 2015/176955 A1 CN 106456029 A | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004006769 A
- JP 2019130070 A